# EUROPEAN PATENT SPECIFICATION

(11) **EP 1 165 544 B1**
(45) Date of publication and mention of the grant of the patent: **06.08.2003**
(21) Application number: 00919742.7
(22) Date of filing: 28.03.2000
(51) Int. Cl.: C07D 401/12, C07D 231/54, C07D 403/12, C07D 495/04, A61K 31/416, A61P 35/00, A61P 43/00

(54) **SUBSTITUTED 1,4-DIHYDROINDENO[1,2-C]PYRAZOLES AS INHIBITORS OF TYROSINE KINASE**
SUBSTITUIERTE 1,4-DIHYDROINDENO[1,2-C]PYRAZOLE ALS INHIBITOREN VON TYROSINKINASE
1,4-DIHYDROINDENO[1,2-C]PYRAZOLES SUBSTITUES UTILISES COMME INHIBITEURS DE LA TYROSINE KINASE

(30) Priority: 06.04.1999 US 127963 P
(43) Date of publication of application: 02.01.2002
(73) Proprietor: Knoll GmbH, 67061 Ludwigshafen (DE)
(72) Inventor: DOYLE, Kevin, J., Nottingham NG1 1GF (GB); RAFFERTY, Paul, Nottingham NG1 1GF (GB); STEELE, Robert, W., Nottingham NG1 1 GF (GB); TURNER, Allyson, Nottingham NG1 1GF (GB); WILKINS, David, J., Nottingham NG1 1GF (GB); ARNOLD, Lee, D., Westborough, MA 01581 (US)
(74) Representative: Modiano, Guido, Dr.-Ing.
(86) International application number: US0008192
(87) International publication number: WO00059901

(56) References cited:
- WO-A-00/27822
- WO-A-99/17770
- US-A- 5 397 787

## Description

This invention relates to certain 3-aryl pyrazoles with 4,5(3,4)-bicyclic ring fusion which are inhibitors of protein kinases particularly tyrosine kinases and serine/threonine kinases, of which some are novel compounds, to pharmaceutical compositions containing these pyrazoles and to processes for preparing these pyrazoles.

There are at least 400 enzymes identified as protein kinases. These enzymes catalyze the phosphorylation of target protein substrates. The phosphorylation is usually a transfer reaction of a phosphate group from ATP to the protein substrate. The specific structure in the target substrate to which the phosphate is transferred is a tyrosine, serine or threonine residue. Since these amino acid residues are the target structures for the phosphoryl transfer, these protein kinase enzymes are commonly referred to as tyrosine kinases or serine/threonine kinases.

The phosphorylation reactions, and counteracting phosphatase reactions, at the tyrosine, serine and threonine residues are involved in countless cellular processes that underlie responses to diverse intracellular signals (typically mediated through cellular receptors), regulation of cellular functions, and activation or deactivation of cellular processes. A cascade of protein kinases often participate in intracellular signal transduction and are necessary for the realization of these cellular processes. Because of their ubiquity in these processes, the protein kinases can be found as an integral part of the plasma membrane or as cytoplasmic enzymes or localized in the nucleus, often as components of enzyme complexes. In many instances, these protein kinases are an essential element of enzyme and structural protein complexes that determine where and when a cellular process occurs within a cell.

### Protein Tyrosine Kinases.

Protein tyrosine kinases (PTKs) are enzymes which catalyse the phosphorylation of specific tyrosine residues in cellular proteins. This post-translational modification of these substrate proteins, often enzymes themselves, acts as a molecular switch regulating cell proliferation, activation or differentiation (for review, see Schlessinger and Ulrich, 1992, Neuron 9:383-391). Aberrant or excessive PTK activity has been observed in many disease states including benign and malignant proliferative disorders as well as diseases resulting from inappropriate activation of the immune system (e.g., autoimmune disorders), allograft rejection, and graft vs. host disease. In addition, endothelial-cell specific receptor PTKs such as KDR and Tie-2 mediate the angiogenic process, and are thus involved in supporting the progression of cancers and other diseases involving inappropriate vascularization (e.g., diabetic retinopathy, choroidal neovascularization due to age-related macular degeneration, psoriasis, arthritis, infantile hemangiomas).

Tyrosine kinases can be of the receptor-type (having extracellular, transmembrane and intracellular domains) or the non-receptor type (being wholly intracellular).

### Receptor Tyrosine Kinases (RTKs).

The RTKs comprise a large family of transmembrane receptors with diverse biological activities. At present, at least nineteen (19) distinct RTK subfamilies have been identified. The receptor tyrosine kinase (RTK) family includes receptors that are crucial for the growth and differentiation of a variety of cell types (Yarden and Ullrich, Ann. Rev. Biochem. 57:433-478, 1988; Ullrich and Schlessinger, Cell 61:243-254, 1990). The intrinsic function of RTKs is activated upon ligand binding, which results in phosphorylation of the receptor and multiple cellular substrates, and subsequently in a variety of cellular responses (Ullrich & Schlessinger, 1990, Cell 61:203-212). Thus, receptor tyrosine kinase mediated signal transduction is initiated by extracellular interaction with a specific growth factor (ligand), typically followed by receptor dimerization, stimulation of the intrinsic protein tyrosine kinase activity and receptor trans-phosphorylation. Binding sites are thereby created for intracellular signal transduction molecules and lead to the formation of complexes with a spectrum of cytoplasmic signaling molecules that facilitate the appropriate cellular response. (e.g., cell division, differentiation, metabolic effects, changes in the extracellular microenvironment) (see Schlessinger and Ullrich, 1992, Neuron 9:1-20).

Proteins with SH2 (src homology -2) or phosphotyrosine binding (PTB) domains bind activated tyrosine kinase receptors and their substrates with high affinity to propagate signals into cells. Both of the domains recognize phosphotyrosine (Fantl et al., 1992, Cell 69:413-423; Songyang et al., 1994, Mol. Cell. Biol. 14:2777-2785; Songyang et al., 1993, Cell 72:767-778; Koch et al., 1991, Science 252:668-678; Shoelson, Curr. Opin. Chem. Biol. (1997), 1(2), 227-234; and Cowburn, Curr. Opin. Strict, Biol. (1997), 7(6), 835-838). Several intracellular substrate proteins that associate with receptor tyrosine kinases (RTKs) have been identified. They may be divided into two principal groups: (1) substrates which have a catalytic domain; and (2) substrates which lack such a domain but serve as adapters and associate with catalytically active molecules (Songyang et al., 1993, Cell 72:767-778). The specificity of the interactions between receptors or proteins and SH2 or PTB domains of their substrates is determined by the amino acid residues immediately surrounding the phosphorylated tyrosine residue. For example, differences in the binding affinities between SH2 domains and the amino acid sequences surrounding the phosphotyrosine residues on particular receptors correlate with the observed differences in their substrate phosphorylation profiles (Songyang et al., 1993, Cell 72:767-778). Observations suggest that the function of each receptor tyrosine kinase is determined not only by its pattern of expression and ligand availability but also by the array of downstream signal transduction pathways that are activated by a particular receptor as well as the timing and duration of those stimuli. Thus, phosphorylation provides an important regulatory step which determines the selectivity of signaling pathways recruited by specific growth factor receptors, as well as differentiation factor receptors.

Several receptor tyrosine kinases, and growth factors that bind thereto, have been suggested to play a role in angiogenesis, although some may promote angiogenesis indirectly (Mustonen and Alitalo, J. Cell Biol. 129:895-898, 1995). One such receptor tyrosine kinase, known as "fetal liver kinase 1" (FLK-1), is a member of the type III subclass of RTKs. An alternative designation for human FLK-1 is "kinase insert domain-containing receptor" (KDR) (Terman et al., Oncogene 6:1677-83, 1991). Another alternative designation for FLK-1/KDR is "vascular endothelial cell growth factor receptor 2" (VEGFR-2) since it binds VEGF with high affinity. The murine version of FLK-1/VEGFR-2 has also been called NYK (Oelrichs et al, Oncogene 8(1):11-15, 1993). DNAs encoding mouse, rat and human FLK-1 have been isolated, and the nucleotide and encoded amino acid sequences reported (Matthews et al., Proc. Natl. Acad. Sci. USA, 88:9026-30, 1991; Terman et al., 1991, supra; Terman et al., Biochem. Biophys. Res. Comm. 187:1579-86, 1992; Sarzani et al., supra; and Millauer et al., Cell 72:835-846, 1993). Numerous studies such as those reported in Millauer et al., supra, suggest that VEGF and FLK-1/KDR/VEGFR-2 are a ligand-receptor pair that play an important role in the proliferation of vascular endothelial cells, and formation and sprouting of blood vessels, termed vasculogenesis and angiogenesis, respectively.

Another type III subclass RTK designated "fins-like tyrosine kinase-1" (Flt-1) is related to FLK-1/KDR (DeVries et al. Science 255;989-991, 1992; Shibuya et al., Oncogene 5:519-524, 1990). An alternative designation for Flt-1 is "vascular endothelial cell growth factor receptor 1" (VEGFR-1). To date, members of the FLK-1/KDR/VEGFR-2 and Flt-1/ VEGFR-1 subfamilies have been found expressed primarily on endothelial cells. These subclass members are specifically stimulated by members of the vascular endothelial cell growth factor (VEGF) family of ligands (Klagsbum and D'Amore, Cytokine & Growth Factor Reviews 7: 259-270, 1996). Vascular endothelial cell growth factor (VEGF) binds to Flt-1 with higher affinity than to FLK-1/KDR and is mitogenic toward vascular endothelial cells (Terman et al., 1992, supra; Mustonen et al. supra; DeVries et al., supra). Flt-1 is believed to be essential for endothelial organization during vascular development. Flt-1 expression is associated with early vascular development in mouse embryos, and with neovascularization during wound healing (Mustonen and Alitalo, supra). Expression of Flt-1 in adult organs such as kidney glomeruli suggests an additional function for this receptor that is not related to cell growth (Mustonen and Alitalo, supra).

As previously stated, recent evidence suggests that VEGF plays a role in the stimulation of both normal and pathological angiogenesis (Jakeman et al., Endocrinology 133: 848-859, 1993; Kolch et al., Breast Cancer Research and Treatment 36: 139-155, 1995; Ferrara et al., Endocrine Reviews 18(1); 4-25, 1997; Ferrara et al., Regulation of Angiogenesis (ed. L. D. Goldberg and E.M. Rosen), 209-232, 1997). In addition, VEGF has been implicated in the control and enhancement of vascular permeability (Connolly, et al., J. Biol. Chem. 264: 20017-20024, 1989; Brown et al., Regulation of Angiogenesis (ed. L.D. Goldberg and E.M. Rosen), 233-269, 1997).

Different forms of VEGF arising from alternative splicing of mRNA have been reported, including the four species described by Ferrara et al. (J. Cell. Biochem. 47:211-218, 1991). Both secreted and predominantly cell-associated species of VEGF have been identified by Ferrara et al. supra, and the protein is known to exist in the form of disulfide linked dimers.

Several related homologs of VEGF have recently been identified. However, their roles in normal physiological and disease processes have not yet been elucidated. In addition, the members of the VEGF family are often coexpressed with VEGF in a number of tissues and are, in general, capable of forming heterodimers with VEGF. This property likely alters the receptor specificity and biological effects of the heterodimers and further complicates the elucidation of their specific functions as illustrated below (Korpelainen and Alitalo, Curr. Opin. Cell Biol., 159-164, 1998 and references cited therein).

Placenta growth factor (P1GF) has an amino acid sequence that exhibits significant homology to the VEGF sequence (Park et al., J. Biol. Chem. 269:25646-54, 1994; Maglione et al. Oncogene 8:925-31, 1993). As with VEGF, different species of P1GF arise from alternative splicing of mRNA, and the protein exists in dimeric form (Park et al., supra). P1GF-1 and P1GF-2 bind to Flt-1 with high affinity, and P1GF-2 also avidly binds to neuropilin-1 (Migdal et al,J. Biol. Chem, 273 (35): 22272-22278), but neither binds to FLK-1/KDR (Park et al., supra). P1GF has been reported to potentiate both the vascular permeability and mitogenic effect of VEGF on endothelial cells when VEGF is present at low concentrations (purportedly due to heterodimer formation) (Park et al., supra).

VEGF-B is produced as two isoforms (167 and 185 residues) that also appear to bind Flt-1/VEGFR-1. It may play a role in the regulation of extracellular matrix degradation, cell adhesion, and migration through modulation of the expression and activity of urokinase type plasminogen activator and plasminogen activator inhibitor 1 (Pepper et al, Proc. Natl. Acad. Sci. U. S. A. (1998), 95(20): 11709-11714).

VEGF-C was originally cloned as a ligand for VEGFR-3/Flt-4 which is primarily expressed by lymphatic endothelial cells. In its fully processed form, VEGF-C can also bind KDR/VEGFR-2 and stimulate proliferation and migration of endothelial cells in vitro and angiogenesis in in vivo models (Lymboussaki et al, Am. J. Pathol. (1998), 153(2): 395-403; Witzenbichler et al, Am. J. Pathol. (1998), 153(2), 381-394). The transgenic overexpression of VEGF-C causes proliferation and enlargement of only lymphatic vessels, while blood vessels are unaffected. Unlike VEGF, the expression of VEGF-C is not induced by hypoxia (Ristimaki et al, J. Biol. Chem. (1998), 273(14),8413-8418).

The most recently discovered VEGF-D is structurally very similar to VEGF-C. VEGF-D is reported to bind and activate at least two VEGFRs, VEGFR-3/Flt-4 and KDR/VEGFR-2. It was originally cloned as a c-fos inducible mitogen for fibroblasts and is most prominently expressed in the mesenchymal cells of the lung and skin (Achen et al, Proc. Natl. Acad. Sci. U. S. A. (1998), 95(2), 548-553 and references therein).

VEGF-C and VEGF-D have been claimed to induce increases in vascular permeability in vivo in a Miles assay when injected into cutaneous tissue (PCT/US97/14696; WO98/07832, Witzenbichler et al., supra). The physiological role and significance of these ligands in modulating vascular hyperpermeability and endothelial responses in tissues where they are expressed remains uncertain.

Based upon emerging discoveries of other homologs of VEGF and VEGFRs and the precedents for ligand and receptor heterodimerization, the actions of such VEGF homologs may involve formation of VEGF ligand heterodimers, and/or heterodimerization of receptors, or binding to a yet undiscovered VEGFR (Witzenbichler et al., supra). Also, recent reports suggest neuropilin-1 (Migdal et al, supra) or VEGFR-3/Flt-4 (Witzenbichler et al., supra), or receptors other than KDR/VEGFR-2 may be responsible for the induction of vascular permeability (Stacker, S.A., Vitali, A., Domagala, T., Nice, E., and Wilks, A.F., "Angiogenesis and Cancer" Conference, Amer. Assoc. Cancer Res., Jan. 1998, Orlando, FL; Williams, Diabetelogia 40: S118-120 (1997)). Until now, no direct evidence for the essential role of KDR in VEGF-mediated vascular hyperpermeability has been disclosed.

### The Non-Receptor Tyrosine Kinases.

The non-receptor tyrosine kinases represent a collection of cellular enzymes which lack extracellular and transmembrane sequences. At present, over twenty-four individual non-receptor tyrosine kinases, comprising eleven (11) subfamilies (Src, Frk, Btk, Csk, Abl, Zap70, Fes/Fps, Fak, Jak, Ack and LIMK) have been identified. At present, the Src subfamily of non-receptor tyrosine kinases is comprised of the largest number of PTKs and include Src, Yes, Fyn, Lyn, Lck, Blk, Hck, Fgr and Yrk. The Src subfamily of enzymes has been linked to oncogenesis. A more detailed discussion of non-receptor tyrosine kinases is provided in Bolen, 1993, Oncogene 8:2025-2031.

Many of the tyrosine kinases, whether an RTK or non-receptor tyrosine kinase, have been found to be involved in cellular signaling pathways involved in numerous pathogenic conditions, including cancer, psoriasis, and other hyperproliferative disorders or hyper-immune responses.

### Development of Compounds to Modulate the PTKs.

In view of the surmised importance of PTKs to the control, regulation, and modulation of cell proliferation, the diseases and disorders associated with abnormal cell proliferation, many attempts have been made to identify receptor and non-receptor tyrosine kinase "inhibitors" using a variety of approaches, including the use of mutant ligands (U.S. Patent No. 4,966,849), soluble receptors and antibodies (Application No. WO 94/10202; Kendall & Thomas, 1994, Proc. Natl. Acad. Sci 90:10705-09; Kim et al., 1993, Nature 362:841-844), RNA ligands (Jellinek, et al., Biochemistry 33:10450-56; Takano, et al., 1993, Mol. Bio. Cell 4:358A; Kinsella, et al. 1992, Exp. Cell Res. 199:56-62; Wright, et al., 1992, J. Cellular Phys. 152:448-57) and tyrosine kinase inhibitors (WO 94/03427; WO 92/21660; WO 91/15495; WO 94/14808; U.S. Patent No. 5,330,992; Mariani, et al., 1994, Proc. Am. Assoc. Cancer Res. 35:2268).

More recently, attempts have been made to identify small molecules which act as tyrosine kinase inhibitors. For example, bis monocyclic, bicyclic or heterocyclic aryl compounds (PCT WO 92/20642) and vinylene-azaindole derivatives (PCT WO 94/14808) have been described generally as tyrosine kinase inhibitors. Styryl compounds (U.S. Patent No. 5,217,999), styryl-substituted pyridyl compounds (U.S. Patent No. 5,302,606), certain quinazoline derivatives (EP Application No. 0 566 266 A1; Expert Opin. Ther. Pat. (1998), 8(4): 475-478), selenoindoles and selenides (PCT WO 94/03427), tricyclic polyhydroxylic compounds (PCT WO 92/21660) and benzylphosphonic acid compounds (PCT WO 91/15495) have been described as compounds for use as tyrosine kinase inhibitors for use in the treatment of cancer. Anilinocinnolines (PCT WO97/34876) and quinazoline derivative compounds (PCT WO97/22596; PCT WO97/42187) have been described as inhibitors of angiogenesis and vascular permeability.

In addition, attempts have been made to identify small molecules which act as serine/threonine kinase inhibitors. In particular, bis(indolylmaleimide) compounds have been described as inhibiting particular PKC serine/threonine kinase isoforms whose dysfunction is associated with altered vascular permeability in VEGF-related diseases (PCT WO97/40830; PCT WO97/40831).

The identification of effective small compounds which specifically inhibit signal transduction by modulating the activity of receptor and non-receptor tyrosine and serine/threonine kinases to regulate and modulate abnormal or inappropriate cell proliferation, differentiation, or metabolism is therefore desirable. In particular, the identification of methods and compounds that specifically inhibit the function of a tyrosine kinase which is essential for angiogenic processes or the formation of vascular hyperpermeability leading to edema, ascites, effusions, exudates, and macromolecular extravasation and matrix deposition as well as associated disorders would be beneficial.

### SUMMARY OF THE INVENTION

The present invention provides compounds of formula I 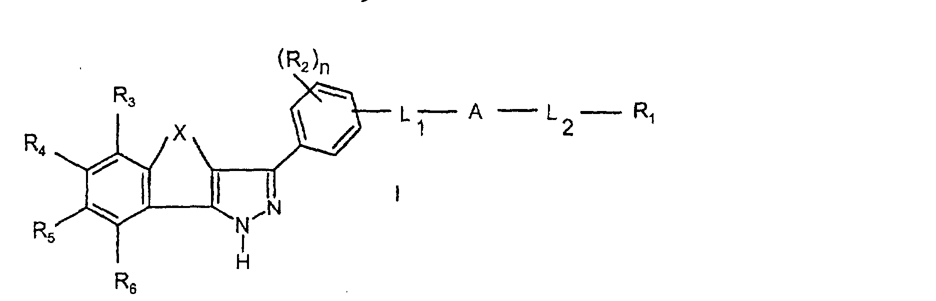 and pharmaceutically acceptable salts thereof in which
L₁ represents a group of formula (E)ₛ(CH₂)_{q} in which E represents NR₂₄, O or S, s is 0 or 1 and q is an integer from 0 to 6, provided that when s is 1 q is at least 1, in which the alkylene chain is optionally substituted by one or more of the following: a C₁₋₆ alkyl group optionally substituted by one or more hydroxy, halo or optionally substituted amino; a C₁₋₆ alkoxy group optionally substituted by one or more hydroxy, halo or optionally substituted amino; hydroxy; halo; or optionally substituted amino;
A represents CONH, NHCO, SO₂NH, NHSO₂, or NR₂₅ ; L₂ represents a group of formula (CH₂)ᵣ in which r is an integer from 0 to 6 in which the alkylene chain is optionally substituted by one or more of the following: a C₁₋₆ alkyl group optionally substituted by one or more hydroxy, halo or optionally substituted amino; a C₁₋₆ alkoxy group optionally substituted by one or more hydroxy, halo or optionally substituted amino; hydroxy; halo; or optionally substituted amino;
R₂ represents a C₁₋₆ alkyl group optionally substituted by one or more of the following: halo, hydroxy, C₁₋₆ alkoxy or optionally substituted amino; a C₁₋₆ alkoxy group optionally substituted by one or more of the following: halo, hydroxy, C₁₋₆ alkoxy or optionally substituted amino ; or R₂ is halo, hydroxy, cyano, nitro, carbamoyl, a C₁₋₆ alkanoylgroup, a (C₁₋₆ alkoxy)carbonyl group or optionally substituted amino;
n represents 0,1,2 or 3
X represents a) substituted methylene b) carbonyl, c) oxygen , d) a group of formula - C=NOR₇ in which R₇ represents H or a C₁₋₄ alkyl group, e) a group of formula NR₈ in which R₈ represents H, an optionally substituted C₁₋₄ alkyl group or optionally substituted phenyl, f) a group of formula (CH₂)ₙ in which n is 1, 2 or 3, or g) a group of formula S(O)ₚ in which p is 0, 1 or 2;
R₃, R₄, R₅ and R₆ independently represent a) H, b) halo, c) a C₁₋₆ alkyl group optionally substituted by one or more of the following: hydroxy, a C₁₋₆ alkoxy group, halo or an optionally substituted amino group d) a C₁₋₆ alkoxy group optionally substituted by one or more of the following: hydroxy; a C₁₋₆ alkoxy group; halo; or an optionally substituted amino group provided that these groups are not attached to the carbon which is attached to the oxygen of the alkoxy group; e) optionally substituted phenoxy, f) hydroxy, g) a group formula CORₐ in which Rₐ represents hydroxy, a C₁₋₆ alkoxy group or Rₐ represents an optionally substituted amino group, h) an optionally substituted amino group i) a C₁₋₆ alkanoyl group j) nitro, k) optionally substituted phenyl C₁₋₆ alkyl, 1) optionally substituted phenyl C₁₋₆ alkoxy m) cyano ; or o) a C₂₋₄ alkenyl group or a C₂₋₄ alkynyl group each of which is optionally substituted by phenyl which is optionally substituted by one or more of the following : a C₁₋₆ alkyl group, a C₁₋₆ alkoxy group or halo;
and
1) when A is SO₂NH, or NHSO₂
   R₁ represents a) optionally substituted phenyl b) optionally substituted heteroaryl, c) a five, six or seven membered saturated heterocyclic ring containing a nitrogen atom which optionally contains an additional hetero atom selected from O, S or N and is optionally substituted by a C₁₋₆ alkyl group wherein said saturated ring may be attached through carbon or a hetero atom d) an optionally substituted amino group or e) a C₁₋₆ alkoxy group;
2) when A represents CONH or NHCO
   R₁ represents a) phenyl substituted by nitro or one or more C₁₋₆ alkoxy groups optionally substituted by one or more of the following: halo, hydroxy, C₁₋₆ alkoxy or optionally substituted amino b) optionally substituted heteroaryl or c) a five, six or seven membered saturated heterocyclic ring containing a nitrogen atom which optionally contains an additional hetero atom selected from O, S or N and is optionally substituted by a C₁₋₆ alkyl group wherein said saturated ring is attached through a carbon atom or d) a C₁₋₆ alkoxy group;
3) when A represents a group NR₂₅ and q is at least 1 then
   R₁ represents a) optionally substituted phenyl b) optionally substituted heteroaryl or c) an optionally substituted amino group; and
4) when A represents a group NR₂₅ and q is 0 and s is 0 then R₁ represents optionally substituted heteroaryl;
   R₂₄ and R₂₅ independently represent H; a C₁₋₆ alkyl group optionally substituted by one or more of the following: hydroxy, a C₁₋₆ alkoxy group, halo or an optionally substituted amino group; a C₁₋₆ alkanoyl group or a C₁₋₆ alkylsulphonyl group; provided that no two hetero atoms are attached to the same sp3 hybridized carbon atom.

It will be appreciated by those skilled in the art that when n is other than 0 or 1 that the groups R₂ may be the same or different.

The term optionally substituted amino group means a group of formula NRₖRₗ in which Rₖ and Rₗ independently represent hydrogen, a C₁₋₁₂ alkyl group, a C₁₋₁₂ alkoxy group, a C₃₋₁₂ cycloalkyl group, a phenyl group, a phenylC₁₋₆ alkyl group, a heteroaryl group or a heteroarylC₁₋₆ alkyl group wherein each of said groups is optionallly substituted by one or more of the following: a C₁₋₆ alkyl group, a C₁₋₆ alkoxy group, hydroxy, halo; or Rₖ and Rₗ together with the nitrogen atom to which they are attached represent a five, six or seven membered saturated heterocyclic ring which optionally contains an additional hetero atom selected from 0, S or N and is optionally substituted by a C₁₋₆ alkyl group.

The term optionally substituted as used herein with reference to phenyl and heteroaryl means substituted by one or more of the following a) halo, b) a C₁₋₆ alkyl group optionally substituted by one or more of the following: hydroxy, halo, an optionally substituted amino group or a five, six or seven membered saturated heterocyclic ring containing a nitrogen atom which optionally contains an additional hetero atom selected from O, S or N and is optionally substituted by a C₁₋₆ alkyl group wherein said saturated ring is attached through a carbon atom, c) a C₁₋₆ alkoxy group optionally substituted by one or more of the following: hydroxy; a C₁₋₆ alkoxy group; halo; or optionally substituted amino group provided that these groups are not attached to the carbon which is attached to the oxygen of the alkoxy group, or a five, six or seven membered saturated heterocyclic ring containing a nitrogen atom which optionally contains an additional hetero atom selected from O, S or N and is optionally substituted by a C₁₋₆ alkyl group wherein said saturated ring is attached through a carbon atom, d) optionally substituted phenoxy, e) hydroxy, f) a group formula CORₐ in which Rₐ represents hydroxy, a C₁₋₆ alkoxy group or Rₐ represents a group of formula NR_{b} R_{c} in which R_{b} and R_{c} independently represent hydrogen, a C₁₋₁₂ alkyl group , a C₃₋₁₂ cycloalkyl group or phenyl wherein the alkyl group, the cycloalkyl group and phenyl are optionally substituted by one or more of the following: hydroxy , halo, a C₃₋₁₂ cycloalkyl group or an amino group of formula NRₕRⱼ wherein Rₕ and Rⱼ independently represent hydrogen or a C₁₋₆ alkyl group or wherein Rₕ and Rⱼ together with the nitrogen atom to which they are attached represent a five, six or seven membered saturated heterocyclic ring which optionally contains an additional hetero atom selected from O, S or N and is optionally substituted by a C₁₋₆ alkyl group, g) a group of formula NR_{d} Rₑ in which R_{d} and Rₑ are independently selected from hydrogen, a C₁₋₁₂ alkyl group , a C₃₋₁₂ cycloalkyl group or phenyl or a group of formula COR_{f} wherein R_{f} represents hydrogen, a C₁₋ ₁₂ alkyl group , a C₃₋₁₂ cycloalkyl group, a phenyl C₁₋₆ alkyl group or phenyl wherein in each case the alkyl group, the cycloalkyl group and phenyl are optionally substituted by one or more of the following: halo, hydroxy, nitro or an amino group of formula NRₕRⱼ wherein Rₕ and Rⱼ are as defined above, h) a group of formula O(CH₂)ₘ R_{g} in which m is 2, 3, 4 or 5 and R_{g} represents hydroxy or a group of formula NR_{d}Rₑ in which R_{d} and Rₑ are as defined above; or R_{g} represents a group of formula CORₐ wherein Rₐ is as defined above and m is 1, 2, 3, 4 or 5, i) nitro, j) optionally substituted phenyl C₁₋₆ alkyl, k) optionally substituted phenyl C₁₋₆ alkoxy, 1) cyano, m) a C₃₋₆alkenyloxy group, n) a pyridyloxy or pyridylthio group in which the pyridine ring is optionally substituted by one or more of the following : trifluoromethyl or nitro, o) hydroxyamidino, p) aminomethyl, q) formamidomethyl, r) a C₁₋₆ alkythio group, s) phenyl or t) a C₂₋₄ alkenyl group or a C₂₋₄ alkynyl group each of which is optionally substituted by phenyl which is optionally substituted by one or more of the following : a C₁₋₆ alkyl group, a C₁₋₆ alkoxy group or halo.

The term heteroaryl means an optionally substituted mono or bicyclic aromatic heterocycle in which the heterocycle contains 1, 2, 3 or 4 hetero atoms selected from nitrogen, sulphur or oxygen. The heteroaryl group may be attached through carbon or a hetero atom. Suitable heteroaryl groups include imidazolyl, furyl, pyrrolyl, thienyl, oxazolyl, thiazolyl, isoxazolyl, thiadiazolyl, oxadiazolyl, pyridinyl, pyrimidinyl, pyrazinyl, pyridazinyl, quinolyl, isoquinolyl, indazolyl, benzoxazolyl, benzofuryl, benzothiazolyl, indolizinyl, imidazopyridinyl or benzo(b)thienyl each of which is optionally substituted as described above.

When the optionally substituted amino group represents a saturated heterocyclic ring the ring is preferably morpholino, perhydrothiazin-4-yl, piperidino, pyrrolidin-1-yl, piperazin-1-yl, 4-methylpiperazin-4-yl, thiamorpholinyl, perhydro-1,4-diazepin-1-yl or perhydroazepinyl.

The term substituted methylene means methylene substituted by one or more of the following: hydroxy or a C₁₋₄ alkyl group wherein the alkyl group is optionally further substituted by a group of formula NRᵣRₛ wherein Rᵣ and Rₛ independently represent H or a C₁₋₆ alkyl group.

It will be understood that any group mentioned herein which contains a chain of three or more atoms signifies a group in which the chain may be straight or branched. For example, an alkyl group may comprise propyl, which includes n-propyl and isopropyl, and butyl, which includes n-butyl, sec-butyl, isobutyl and tert-butyl. The term C₃₋₁₂ cycloalkyl group includes bridged groups for example adamantyl. The term 'halo' as used herein signifies fluoro, chloro, bromo and iodo.

Preferably X is CH₂ or S.

In a first group of preferred compounds of formula I, X is CH₂ and A is NR₂₅.

In a second group of preferred compounds of formula I, X is S and A is NR₂₅.

In a third group of preferred compounds of formula I, X is CH₂ and A is HNSO_{2.}

In a fourth group of preferred compounds of formula I, X is CH₂ and A is SO₂NH.

In a fifth group of preferred compounds of formula I, X is CH₂ and A is CONH.

In a sixth group of preferred compounds of formula I, X is CH₂ and A is HNCO.

In most preferred compounds of formula I, X is CH₂, A is NR₂₅ , L₁ is (CH₂)_{q} in which q is an integer from 1 to 6 and the alkylene chain is optionally substituted by one or more of the following: a C₁₋₆ alkyl group optionally substituted by one or more hydroxy, halo or optionally substituted amino; a C₁₋₆ alkoxy group optionally substituted by one or more hydroxy, halo or optionally substituted amino; hydroxy; halo; or optionally substituted amino; L₂ is a bond and R₁ is optionally substituted pyridyl.

Compounds of formula I may exist as salts with pharmaceutically acceptable acids. The present invention includes such salts. Examples of such salts include hydrochlorides, hydrobromides, sulphates, methanesulphonates, nitrates, maleates, acetates, citrates, fumarates, tartrates [eg (+)-tartrates, (-)-tartrates or mixtures thereof including racemic mixtures], succinates, benzoates and salts with amino acids such as glutamic acid. These salts may be prepared by methods known to those skilled in the art.

Certain compounds of formula I which have acidic substituents may exist as salts with pharmaceutically acceptable bases. The present invention includes such salts. Example of such salts include sodium salts, potassium salts, lysine salts and arginine salts. These salts may be prepared by methods known to those skilled in the art.

Certain compounds of formula I and their salts may exist in more than one crystal form and the present invention includes each crystal form and mixtures thereof.

Certain compounds of formula I and their salts may also exist in the form of solvates, for example hydrates, and the present invention includes each solvate and mixtures thereof.

Certain compounds of formula I may contain one or more chiral centres, and exist in different optically active forms, When compounds of formula I contain one chiral centre, the compounds exist in two enantiomeric forms and the present invention includes both enantiomers and mixtures of enantiomers. The enantiomers may be resolved by methods known to those skilled in the art, for example by formation of diastereoisomeric salts which may be separated, for example, by crystallization; formation of diastereoisomeric derivatives or complexes which may be separated, for example, by crystallization, gas-liquid or liquid chromatography; selective reaction of one enantiomer with an enantiomer-specific reagent, for example enzymatic esterification; or gas-liquid or liquid chromatography in a chiral environment, for example on a chiral support for example silica with a bound chiral ligand or in the presence of a chiral solvent. It will be appreciated that where the desired enantiomer is converted into another chemical entity by one of the separation procedures described above, a further step is required to liberate the desired enantiomeric form. Alternatively, specific enantiomers may be synthesized by asymmetric synthesis using optically active reagents, substrates, catalysts or solvents, or by converting one enantiomer into the other by asymmetric transformation.

When a compound of formula I contains more than one chiral centre it may exist in diastereoisomeric forms. The diastereoisomeric pairs may be separated by methods known to those skilled in the art, for example chromatography or crystallization and the individual enantiomers within each pair may be separated as described above. The present invention includes each diastereoisomer of compounds of formula I and mixtures thereof.

Certain compounds of formula I may exist in different tautomeric forms or as different geometric isomers, and the present invention includes each tautomer and/or geometric isomer of compounds of formula I and mixtures thereof.

Certain compounds of formula I may exist in different stable conformational forms which may be separable. Torsional asymmetry due to restricted rotation about an asymmetric single bond, for example because of steric hindrance or ring strain, may permit separation of different conformers. The present invention includes each conformational isomer of compounds of formula I and mixtures thereof.

Certain compounds of formula I may exist in zwitterionic form and the present invention includes each zwitterionic form of compounds of formula I and mixtures thereof.

The compounds of this invention are useful as inhibitors of serine/ threonine and tyrosine kinases. In particular, the compounds of this invention are useful as inhibitors of tyrosine kinases that are important in hyperproliferative diseases, especially in the process of angiogenesis. Since these compounds are anti-angiogenic, they are important substances for inhibiting the progression of disease states where angiogenesis is an important component.

Preferred definitions of the substituents are now given.

Preferably R₁ represents optionally substituted phenyl, optionally substituted thienyl, optionally substituted pyridyl, optionally substituted furyl, or optionally substituted pyrrolyl.

More preferably R₁ represents 2-thienyl, 3-thienyl, 2-furyl, 3-furyl, 2-pyridyl, 3-pyridyl or 4-pyridyl each of which is optionally substituted, and optionally mono-, di- and tri-substituted phenyl, wherein the substituents are selected from optionally substituted alkoxy (particularly methoxy, 3-morpholinopropoxy, 2-morpholinoethoxy, 3-carboxypropoxy, carboxymethoxy, 2-carboxyethoxy, 2-carbamoylethoxy, carbamoylmethoxy, 3-carbamoylpropoxy, 2-piperidinoethoxy, 2-(piperazin-1-yl)ethoxy, 2-(pyrrolidin-1-yl)ethoxy, 2-dimethylaminoethoxy, 2-(perhydro-thiazin-4-yl)ethoxy, 3-piperidinapropoxy, 3-(piperazin-1-yl)propoxy, 3-(pyrrolidin-lyl)-propoxy, 3-dimethylaminopropoxy, 3-(perhydrothiazin-4-yl)propoxy), lower alkyl (particularly methyl), halo (particularly fluoro and chloro), aryl (particularly phenyl), hydroxy, aryloxy (particularly phenoxy), arylalkoxy (particularly benzyloxy), di-lower-alkylamino (particularly dimethylamino), polyhalo-lower-alkyl, polyhalo-lower-alkoxy (particularly difluoromethoxy), nitro, cyano, lower-alkylthio (particularly methylthio), carboxy, lower-alkoxycarbonyl (particularly methoxycarbonyl), amido (particularly acetamido and benzamido) and optionally substituted carbamoyl (particularly carbamoyl, *N*-methylcarbamoyl, *N*-phenylcarbamoyl) and a pyridyloxy or pyridylthio group in which the pyridine ring is optionally substituted by one or more of the following: trifluoromethyl or nitro.

Most preferably R₁ represents 4-pyridyl, 2-formamidomethyl-4-pyridyl, 2-aminomethyl-4-pyridyl, 2-(hydroxyamidino)-4-pyridyl, 2-carbamoyl-4-pyridyl, 4-pyridyl-N-oxide, 2-chloro-4-pyridyl, 2-cyano-4-pyridyl, 5-methyl-2-furyl, 5-(2-nitro-4-trifluoromethylphenyl)fur-2-yl, phenyl, 4-methoxyphenyl, 3-methoxyphenyl, 2-methoxyphenyl, 3,4-dimethoxyphenyl, 3,4,5-trimethoxyphenyl, 4-(3-morpholinopropoxy)phenyl, 4-(2-morpholinoethoxy)phenyl, 4-(3-carboxypropoxy)phenyl, 4-carboxymethoxyphenyl, 4-(3-carbamoylpropoxy)phenyl, 4-carbamoylmethoxyphenyl, 3-(3-morpholino-propoxy)phenyl, 3-(2-morpholinoethoxy)phenyl, 3-(3-carboxypropoxy)phenyl, 4-carboxymethoxyphenyl, 3-(3-carbamoylpropoxy)phenyl, 3-carbamoylmethoxyphenyl, 2-hydroxyphenyl, 3-hydroxyphenyl, 4-hydroxyphenyl, 3-hydroxy-4-methoxyphenyl, 4-hydroxy-3-methoxyphenyl, 4-difluoromethoxyphenyl, 3-nitrophenyl, 4-nitrophenyl, 3,5-di-*tert*-butyl-4-hydroxyphenyl, 4-methylphenyl, 4-fluorophenyl, 2-chlorophenyl, 3-chlorophenyl, 4-chlorophenyl, 2,4-dichlorophenyl, 2-chloro-5-nitrophenyl, 4-fluoro-2-chlorophenyl, 4-methylthiophenyl, 4-biphenylyl, 3-phenoxyphenyl, 4-phenoxyphenyl, 4-benzyloxyphenyl, 4-dimethylaminophenyl, 4-diethylaminophenyl, 4-methoxycarbonylphenyl, 4-carbamoylphenyl, 4-cyanophenyl, 4-*N*-methylcarbamoylphenyl, 4-*N*-phenylcarbamoylphenyl, 4-acetamidophenyl, 4-benzamidophenyl , 4-carboxyphenyl, 4-[*N*-(2-diethylaminoethyl)carbamoyl]phenyl, 4-(prop-1-enyloxy)phenyl, 3-(2-hydroxyethoxy)phenyl, 3-(*N*-(2-diethyl aminoethyl)-carbamoylmethoxy)phenyl, 3-[3-(*N*-(2-diethylaminoethyl)carbamoyl) propoxy]phenyl, 4-(*N-*(2-diethylaminoethyl)carbamoylmethoxy)phenyl, 4-[3-(*N*-(2-diethylaminoethyl)-carbamoyl)propoxy]phenyl, 2-furyl, 5-[3,5-bis(trifluoromethyl)phenyl]-2-furyl, 3-bromo-2-thienyl, 5-methoxy-2-furyl, 5-(2-nitro-4-trifluoromethylphenyl)-2-furyl, 3-N-(2-morpholinoethyl)carbamoylmethoxy)phenyl, 3-[3-(*N*-(2-morpholinoethyl) carbamoyl)propoxyphenyl], 4-(*N*-(2-morpholinoethyl)-carbamoylmethoxy)phenyl, 4-(morpholinoacetamido)phenyl and 4-[3-(*N*-(2-morpholinoethyl)carbamoyl)propoxy]-phenyl.

Preferably R₃, R₄, R₅ and R₆ independently represent hydrogen , halo (particularly fluoro), optionally substituted lower alkoxy (particularly methoxy, 3-morpholinopropoxy, 2-morpholinoethoxy, 3-carboxypropoxy, carboxymethoxy, 2-carboxyethoxy, 2-carbamoylethoxy, 3-carbamoylpropoxy, 2-piperidinoethoxy, 2-(piperazin-1-yl)ethoxy, 2-(pyrrolidin-1-yl)ethoxy, 2-dimethylaminoethoxy, 2-(perhydrothiazin-1-yl)ethoxy, 3-piperidinopropoxy, 3-(piperazin-1-yl)propoxy, 3-(pyrro1idin-1-yl)propoxy, 3-dimethylaminopropoxy, 3-(perhydrothiazin-4-yl)propoxy), carbamoylmethoxy, hydroxypropyloxy, hydroxyethoxy, (3-morpholino)propoxy and 2-morpholino)ethoxy), amido (particularly acetamido and benzamido), optionally substituted carbamoyl (particularly carbamoyl, *N*-methylcarbamoyl and *N*-phenylcarbamoyl), carboxy, nitro and amino.

More preferably R₃, R₄, R₅ and R₆ represent the following: 6,7-dimethoxy, 6,7,8-trimethoxy, 6-fluoro, 6-acetamido, 7-methoxy, 6-carbamoyl, 6-(*N*-methylcarbamoyl), 6-(N-phenylcarbamoyl), (3-morpholino)propoxy and 2-morpholino)-ethoxy.

The term lower as used herein means a group having 1 to 6 carbon atoms.

Specific compounds of the present invention include:
4-(1,4-dihydroindeno[1,2-c]pyrazole)-N-(4-pyridyl)benzylamine
N-[4-(1,4-dihydroindeno[1,2-c]pyrazol-3-yl)phenyl]benzenesulphonamide
4-(1,4-dihydroindeno[1,2-c]pyrazol-3-yl)-N-(2-methoxyethyl)benzamide
4-(1,4-dihydroindeno[1,2-c]pyrazol-3-yl)-N-(4-nitrophenyl)benzamide
4-(1,4-dihydroindeno[1,2-c]pyrazol-3-yl)imidazol-1-ylacetanilide
4-(1,4-dihydroindeno[1,2-c]pyrazol-3-yl)-N-[2-(imidazol-1-yl)ethyl]aniline
4-(1,4-dihydroindeno[1,2-c]pyrazol-3-yl)-N-(2-morpholinoethyl) benzenesulphonamide
4-(1,4-dihydroindeno[1,2-c]pyrazol-3-yl)-N-(2-methoxyethyl)benzenesulphonamide
N-[2-(N,N-diethylamino)ethyl]-4-(1,4-dihydroindeno[1,2-c]pyrazol-3-yl)benzylamine
N-[2-(N,N-diethylamino)ethyl]-4-(1,4-dihydroindeno[1,2-c]pyrazol-3-yl)benzenesulphonamide
4-(1,4-dihydroindeno[1,2-c]pyrazol-3-yl)-N-(2-morpholinoethyl)benzylamine
N-(4-ethoxyphenyl)-4-(1,4-dihydroindeno[1,2-c]pyrazol-3-yl)benzylamine
(S)-4-(1,4-dihydroindeno[1,2-c]pyrazol-3-yl)-N-(pyrrolidin-2-ylmethyl)benzamide
4-(1H-[1]benzothieno[3,2-c]pyrazol-3-yl)-N-[3-(imidazol-1-yl)propyl]benzylamine
4-(1H-[1]benzothieno[3,2-c]pyrazol-3-yl)-N-(2-morpholinoethyl)benzylamine
and pharmaceutically acceptable salts thereof including individual enantiomers and mixture of enantiomers.

The present invention further includes the use of these compounds in pharmaceutical compositions with a pharmaceutically effective amount of the above-described compounds and a pharmaceutically acceptable carrier or excipient. These pharmaceutical compositions can be administered to individuals to slow or halt the process of angiogenesis in angiogenesis-aided diseases, or to treat edcma, effusions, exudates, or ascites and other conditions associated with vascular hyperpermeability.

### DETAILED DESCRIPTION OF THE INVENTION

The compounds of this invention have antiangiogenic properties. These antiangiogenic properties are due at least in part to the inhibition of protein tyrosine kinases essential for angiogenic processes. For this reason, these compounds can be. used as active agents against such disease states as arthritis, atherosclerosis, psoriasis, hemangiomas, myocardial angiogenesis, coronary and cerebral collaterals, ischemic limb angiogenesis, wound healing, peptic ulcer Helicobacter related diseases, fractures, cat scratch fever, rubeosis, neovascular glaucoma and retinopathies such as those associated with diabetic retinopathy or age-related macular degeneration. In addition, some of these compounds can be used as active agents against solid tumors, malignant ascites, hematopoietic cancers and hyperproliferative disorders such as thyroid hyperplasia (especially Grave's disease), and cysts (such as hypervascularity of ovarian stroma characteristic of polycystic ovarian syndrome (Stein-Leventhal syndrome)) since such diseases require a proliferation of blood vessel cells for growth and/or metastasis.

Further, some of these compounds can be used as active agents against burns, chronic lung disease, stroke, polyps, anaphylaxis, chronic and allergic inflammation, ovarian hyperstimulation syndrome, brain tumor-associated cerebral edema, high-altitude, trauma or hypoxia induced cerebral or pulmonary edema, ocular and macular edema, ascites, and other diseases where vascular hyperpermeability, effusions, exudates, protein extravasation, or edema is a manifestation of the disease. The compounds will also be useful in treating disorders in which protein extravasation leads to the deposition of fibrin and extracellular matrix, promoting stromal proliferation (e.g. fibrosis, cirrhosis and carpal tunnel syndrome).

VEGF's are unique in that they are the only angiogenic growth factors known to contribute to vascular hyperpermeability and the formation of edema. Indeed, vascular hyperpermeability and edema that is associated with the expression or administration of many other growth factors appears to be mediated via VEGF production. Inflammatory cytokines stimulate VEGF production. Hypoxia results in a marked upregulation of VEGF in numerous tissues, hence situations involving infarct, occlusion, ischemia, anemia, or circulatory impairment typically invoke VEGF/VPF mediated responses. Vascular hyperpermeability, associated edema, altered transendothelial exchange and macromolecular extravasation, which is often accompanied by diapedesis, can result in excessive matrix deposition, aberrant stromal proliferation, fibrosis, etc. Hence, VEGF-mediated hyperpermeability can significantly contribute to disorders with these etiologic features.

It is envisaged that the disorders listed above are mediated to a significant extent by protein tyrosine kinase activity involving the KDR/VEGFR-2 and/or the Flt-1/VEGFR-1 tyrosine kinases. By inhibiting the activity of these tyrosine kinases, the progression of the listed disorders is inhibited because the angiogenic or vascular hyperpermeability component of the disease state is severely curtailed. The action of the compounds of this invention, by their selectivity for specific tyrosine kinases, result in a minimization of side effects that would occur if less selective tyrosine kinase inhibitors were used.

The compounds of this invention have inhibitory activity against protein kinases. That is, these compounds modulate signal transduction by protein kinases. Compounds of this invention inhibit protein kinases from serine/threonine and tyrosine kinase classes. In particular, these compounds selectively inhibit the activity of the KDR/FLK-1/VEGFR-2 tyrosine kinases. Certain compounds of this invention also inhibit the activity of additional tyrosine kinases such as Flt-1/VEGFR-1, Src-subfamily kinases such as Lck, Src, fyn, yes, etc. Additionally, some compounds of this invention significantly inhibit serine/threonine kinases such as CDKs which play an essential role in cell-cycle progression. The potency and specificity of the generic compounds of this invention towards a particular protein kinase can often be altered and optimized by variations in the nature, number and arrangement of the substituents (i.e., R₁, R₂, R₃, R₄, R₅ and R₆) of and conformational restrictions. In addition the metabolites of certain compounds may also possess significant protein kinase inhibitory activity.

The compounds of this invention, when administered to individuals in need of such compounds, inhibit vascular hyperpermeability and the formation of edema in these individuals. These compounds act, it is believed, by inhibiting the activity of KDR tyrosine kinase which is involved in the process of vascular hyperpermeability and edema formation. The KDR tyrosine kinase may also be referred to as FLK-1 tyrosine kinase, NYK tyrosine kinase or VEGFR-2 tyrosine kinase. KDR tyrosine kinase is activated when vascular endothelial cell growth factor (VEGF) or another activating ligand (such as VEGF-C, VEGF-D or HIV Tat protein) binds to a KDR tyrosine kinase receptor which lies on the surface of vascular endothelial cells. Following such KDR tyrosine kinase activation, hyperpermeability of the blood vessels occurs and fluid moves from the blood stream past the blood vessel walls into the interstitial spaces, thereby forming an area of edema. Diapedesis also often accompanies this response, Similarly, excessive vascular hyperpermeability can disrupt normal molecular exchange across the endothelium in critical tissues and organs (e.g., lung and kidney), thereby causing macromolecular extravasation and deposition. Following this acute response to KDR stimulation which is believed to facilitate the subsequent angiogenic process, prolonged KDR tyrosine kinase stimulation results in the proliferation and chemotaxis of vascular endothelial cells and formation of new vessels. By inhibiting KDR tyrosine kinase activity, either by blocking the production of the activating ligand, by blocking the activating ligand binding to the KDR tyrosine kinase receptor, by preventing receptor dimerization and transphosphorylation, by inhibiting the enzyme activity of the KDR tyrosine kinase (inhibiting the phosphorylation function of the enzyme) or by some other mechanism that interrupts its downstream signaling (D. Mukhopedhyay *et al*., *Cancer Res*. 58:1278-1284 (1998) and references therein), hyperpermeability, as well as associated extravasation, subsequent edema formation and matrix deposition, and angiogenic responses, may be inhibited and minimized.

One group of preferred compounds of this invention has the property of inhibiting KDR tyrosine kinase activity without significantly inhibiting Flt-1 tyrosine kinase activity (Flt-1 tyrosine kinase is also referred to as VEGFR-1 tyrosine kinase). Both KDR tyrosine kinase and Flt-1 tyrosine kinase are activated by VEGF binding to KDR tyrosine kinase receptors and to Flt-1 tyrosine kinase receptors, respectively. Since Flt-1 tyrosine kinase activity may mediate important events in endothelial maintenance and vascular function, an inhibition of this enzyme activity may lead to toxic or adverse effects. At the very least, such inhibition is unnecessary for blocking the angiogenic responses, induction of vascular hyperpermeability and the formation of edema, so it is wasteful and of no value to the individual. Certain preferred compounds of this invention are unique because they inhibit the activity of one VEGF-receptor tyrosine kinase (KDR) that is activated by activating ligands but do not inhibit other receptor tyrosine kinases, such as Flt-1, that are also activated by certain activating ligands. The preferred compounds of this invention are, therefore, selective in their tyrosine kinase inhibitory activity.

The compounds of the present invention are also useful in the treatment of ulcers - bacterial, fungal, Mooren ulcers and ulcerative colitis.

The compounds of the present invention are also useful in the treatment of conditions wherein undesired angiogenesis, edema, or stromal deposition occurs in viral infections such as Herpes simplex, Herpes Zoster, AIDS, Kaposi's sarcoma, protozoan infections and toxoplasmosis, following trauma, radiation, stroke, endometriosis, ovarian hyperstimulation syndrome, systemic lupus, sarcoidosis, synovitis, Crohn's disease, sickle cell anaemia, Lyme's disease, pemphigoid, Paget's disease, hyperviscosity syndrome, Osler-Weber-Rendu disease, chronic inflammation, chronic occlusive pulmonary disease, asthma, rheumatoid arthritis and osteoarthritis.

The compounds of the present invention are also useful in the treatment of ocular conditions such as ocular and macular edema, ocular neovascular disease, scleritis, radial keratotomy, uveitis, vitritis, myopia, optic pits, chronic retinal detachment, post-laser complications, conjunctivitis, Stargardt's disease and Eales disease in addition to retinopathy and macular degeneration.

The compounds of the present invention are also useful in the treatment of cardiovascular conditions such as atherosclerosis, restenosis, vascular occlusion and carotid obstructive disease.

The compounds of the present invention are also useful in the treatment of cancer related indications such as solid tumors, sarcomas (especially Ewing's sarcoma and osteosarcoma), retinoblastoma, rhabdomyosarcomas, neuroblastoma, hematopoietic malignancies, including leukaemia and lymphoma, tumor-induced pleural or pericardial effusions, and malignant ascites.

The compounds of the present invention are also useful in the treatment of diabetic conditions such as glaucoma, diabetic retinopathy and microangiopathy.

It is envisaged that the disorders listed above are mediated to a significant extent by protein tyrosine kinase activity involving the VEGF receptors (e.g. KDR and Flt-1). By inhibiting the activity of these receptor tyrosine kinases, the progression of the listed disorders is inhibited because the angiogenic component of the disease state is severely curtailed. The action of the compounds of this invention, by their selectivity for specific tyrosine kinases, result in a minimization of side effects that would occur if less selective tyrosine kinase inhibitors were used.

In another aspect the present invention provides compounds of formula I as defined initially above (including the provisos) for use as medicaments, particularly as inhibitors of protein kinase activity for example tyrosine kinase activity, serine kinase activity and threonine kinase activity. In yet another aspect the present invention provides the use of compounds of formula I as defined initially above (including the provisos) in the manufacture of a medicament for use in the inhibition of protein kinase activity.

In this invention, the following definitions are applicable:
"Pharmaceutically acceptable salts" refers to those salts which retain the biological effectiveness and properties of the free bases and which are obtained by reaction with inorganic acids such as hydrochloric acid, hydrobromic acid, sulfuric acid, nitric acid, phosphoric acid or organic acids such as sulfonic acid, carboxylic acid, organic phosphoric acid, methanesulfonic acid, ethanesulfonic acid, p-toluenesulfonic acid, salicylic acid, lactic acid, tartaric acid and the like.
"Alkyl" refers to a saturated aliphatic hydrocarbon, including straight-chain and branched-chain groups having 1 to 4 carbons.
"Alkoxy" refers to an "O-alkyl" group, where "alkyl" is defined as described above.

### Pharmaceutical Formulations

The compounds of this invention can be administered to a human patient by themselves or in pharmaceutical compositions where they are mixed with suitable carriers or excipient(s) at doses to treat or ameliorate vascular hyperpermeability, edema and associated disorders. Mixtures of these compounds can also be administered to the patient as a simple mixture or in suitable formulated pharmaceutical compositions. A therapeutically effective dose further refers to that amount of the compound or compounds sufficient to result in the prevention or attenuation of inappropriate neovascularization, progression of hyperproliferative disorders, edema, VEGF-associated hyperpermeability and/or VEGF-related hypotension. Techniques for formulation and administration of the compounds of the instant application may be found in "Remington's Pharmaceutical Sciences," Mack Publishing Co., Easton, PA, latest edition.

### Routes of Administration

Suitable routes of administration may, for example, include oral, eyedrop, rectal, transmucosal, topical, or intestinal administration; parenteral delivery, including intramuscular, subcutaneous, intramedullary injections, as well as intrathecal, direct intraventricular, intravenous, intraperitoneal, intranasal, or intraocular injections.

Alternatively, one may administer the compound in a local rather than a systemic manner, for example, via injection of the compound directly into an edematous site, often in a depot or sustained release formulation.

Furthermore, one may administer the drug in a targeted drug delivery system, for example, in a liposome coated with endothelial cell-specific antibody.

### Composition/Formulation

The pharmaceutical compositions of the present invention may be manufactured in a manner that is itself known, e.g., by means of conventional mixing, dissolving, granulating, dragee-making, levigating, emulsifying, encapsulating, entrapping or lyophilizing processes.

Pharmaceutical compositions for use in accordance with the present invention thus may be formulated in conventional manner using one or more physiologically acceptable carriers comprising excipients and auxiliaries which facilitate processing of the active compounds into preparations which can be used pharmaceutically. Proper formulation is dependent upon the route of administration chosen.

For injection, the agents of the invention may be formulated in aqueous solutions, preferably in physiologically compatible buffers such as Hanks's solution, Ringer's solution, or physiological saline buffer. For transmucosal administration, penetrants appropriate to the barrier to be permeated are used in the formulation. Such penetrants are generally known in the art.

For oral administration, the compounds can be formulated readily by combining the active compounds with pharmaceutically acceptable carriers well known in the art. Such carriers enable the compounds of the invention to be formulated as tablets, pills, dragees, capsules, liquids, gels, syrups, slurries, suspensions and the like, for oral ingestion by a patient to be treated. Pharmaceutical preparations for oral use can be obtained by combining the active compound with a solid excipient, optionally grinding a resulting mixture, and processing the mixture of granules, after adding suitable auxiliaries, if desired, to obtain tablets or dragee cores. Suitable excipients are, in particular, fillers such as sugars, including lactose, sucrose, mannitol, or sorbitol; cellulose preparations such as, for example, maize starch, wheat starch, rice starch, potato starch, gelatin, gum tragacanth, methyl cellulose, hydroxypropylmethyl-cellulose, sodium carboxymethylcellulose, and/or polyvinylpyrrolidone (PVP). If desired, disintegrating agents may be added, such as the cross-linked polyvinyl pyrrolidone, agar, or alginic acid or a salt thereof such as sodium alginate.

Dragee cores are provided with suitable coatings. For this purpose, concentrated sugar solutions may be used, which may optionally contain gum arabic, talc, polyvinyl pyrrolidone, carbopol gel, polyethylene glycol, and/or titanium dioxide, lacquer solutions, and suitable organic solvents or solvent mixtures. Dyestuffs or pigments may be added to the tablets or dragee coatings for identification or to characterize different combinations of active compound doses.

Pharmaceutical preparations which can be used orally include push-fit capsules made of gelatin, as well as soft, sealed capsules made of gelatin and a plasticizer, such as glycerol or sorbitol. The push-fit capsules can contain the active ingredients in admixture with filler such as lactose, binders such as starches, and/or lubricants such as talc or magnesium stearate and, optionally, stabilizers. In soft capsules, the active compounds may be dissolved or suspended in suitable liquids, such as fatty oils, liquid paraffin, or liquid polyethylene glycols. In addition, stabilizers may be added. All formulations for oral administration should be in dosages suitable for such administration.

For buccal administration, the compositions may take the form of tablets or lozenges formulated in conventional manner.

For administration by inhalation, the compounds for use according to the present invention are conveniently delivered in the form of an aerosol spray presentation from pressurized packs or a nebuliser, with the use of a suitable propellant, e.g., dichlorodifluoromethane, trichlorofluoromethane, dichlorotetrafluoroethane, carbon dioxide or other suitable gas. In the case of pressurized aerosol the dosage unit may be determined by providing a valve to deliver a metered amount. Capsules and cartridges of e.g. gelatin for use in an inhaler or insufflator may be formulated containing a powder mix of the compound and a suitable powder base such as lactose or starch.

The compounds may be formulated for parenteral administration by injection, e.g. bolus injection or continuous infusion. Formulations for injection may be presented in unit dosage form, e.g., in ampoules or in multi-dose containers, with an added preservative. The compositions may take such forms as suspensions, solutions or emulsions in oily or aqueous vehicles, and may contain formulatory agents such as suspending, stabilizing and/or dispersing agents.

Pharmaceutical formulations for parenteral administration include aqueous solutions of the active compounds in water-soluble form. Additionally, suspensions of the active compounds may be prepared as appropriate oily injection suspensions. Suitable lipophilic solvents or vehicles include fatty oils such as sesame oil, or synthetic fatty acid esters, such as ethyl oleate or triglycerides, or liposomes. Aqueous injection suspensions may contain substances which increase the viscosity of the suspension, such as sodium carboxymethyl cellulose, sorbitol, or dextran. Optionally, the suspension may also contain suitable stabilizers or agents which increase the solubility of the compounds to allow for the preparation of highly concentrated solutions.

Alternatively, the active ingredient may be in powder form for constitution with a suitable vehicle, e.g., sterile pyrogen-free water, before use.

The compounds may also be formulated in rectal compositions such as suppositories or retention enemas, e.g., containing conventional suppository bases such as cocoa butter or other glycerides.

In addition to the formulations described previously, the compounds may also be formulated as a depot preparation. Such long acting formulations may be administered by implantation (for example subcutaneously or intramuscularly or by intramuscular injection). Thus, for example, the compounds may be formulated with suitable polymeric or hydrophobic materials (for example as an emulsion in an acceptable oil) or ion exchange resins, or as sparingly soluble derivatives, for example, as a sparingly soluble salt.

An example of a pharmaceutical carrier for the hydrophobic compounds of the invention is a cosolvent system comprising benzyl alcohol, a nonpolar surfactant, a water-miscible organic polymer, and an aqueous phase. The cosolvent system may be the VPD co-solvent system. VPD is a solution of 3% w/v benzyl alcohol, 8% w/v of the nonpolar surfactant polysorbate 80, and 65% w/v polyethylene glycol 300, made up to volume in absolute ethanol. The VPD co-solvent system (VPD:5W) consists of VPD diluted 1:1 with a 5% dextrose in water solution. This co-solvent system dissolves hydrophobic compounds well, and itself produces low toxicity upon systemic administration. Naturally, the proportions of a co-solvent system may be varied considerably without destroying its solubility and toxicity characteristics. Furthermore, the identity of the co-solvent components may be varied: for example, other low-toxicity nonpolar surfactants may be used instead of polysorbate 80; the fraction size of polyethylene glycol may be varied; other biocompatible polymers may replace polyethylene glycol, e.g. polyvinyl pyrrolidone; and other sugars or polysaccharides may substitute for dextrose.

Alternatively, other delivery systems for hydrophobic pharmaceutical compounds may be employed. Liposomes and emulsions are well known examples of delivery vehicles or carriers for hydrophobic drugs. Certain organic solvents such as dimethysulfoxide also may be employed, although usually at the cost of greater toxicity. Additionally, the compounds may be delivered using a sustained-release system, such as semipermeable matrices of solid hydrophobic polymers containing the therapeutic agent. Various sustained-release materials have been established and are well known by those skilled in the art. Sustained-release capsules may, depending on their chemical nature, release the compounds for a few weeks up to over 100 days. Depending on the chemical nature and the biological stability of the therapeutic reagent, additional strategies for protein stabilization may be employed.

The pharmaceutical compositions also may comprise suitable solid or gel phase carriers or excipients. Examples of such carriers or excipients include but are not limited to calcium carbonate, calcium phosphate, various sugars, starches, cellulose derivatives, gelatin, and polymers such as polyethylene glycols.

Many of the organic molecule compounds of the invention may be provided as salts with pharmaceutically compatible counterions. Pharmaceutically compatible salts may be formed with many acids, including but not limited to hydrochloric; sulfuric, acetic, lactic, tartaric, malic, succinic, etc. Salts tend to be more soluble in aqueous or other protonic solvents than are the corresponding free base forms.

### Effective Dosage

Pharmaceutical compositions suitable for use in the present invention include compositions wherein the active ingredients are contained in an effective amount to achieve its intended purpose. More specifically, a therapeutically effective amount means an amount effective to prevent development of or to alleviate the existing symptoms of the subject being treated. Determination of the effective amounts is well within the capability of those skilled in the art.

For any compound used in the method of the invention, the therapeutically effective dose can be estimated initially from cellular assays. For example, a dose can be formulated in cellular and animal models to achieve a circulating concentration range that includes the IC₅₀ as determined in cellular assays (i.e., the concentration of the test compound which achieves a half-maximal inhibition of a given protein kinase activity). In some cases it is appropriate to determine the IC₅₀ in the presence of 3 to 5% serum albumin since such a determination approximates the binding effects of plasma protein on the compound. Such information can be used to more accurately determine useful doses in humans. Further, the most preferred compounds for systemic administration effectively inhibit protein kinase signaling in intact cells at levels that are safely achievable in plasma.

A therapeutically effective dose refers to that amount of the compound that results in amelioration of symptoms in a patient. Toxicity and therapeutic efficacy of such compounds can be determined by standard pharmaceutical procedures in cell cultures or experimental animals, e.g., for determining the maximum tolerated dose (MTD) and the ED₅₀ (effective dose for 50% maximal response). The dose ratio between toxic and therapeutic effects is the therapeutic index and it can be expressed as the ratio between MTD and ED₅₀. Compounds which exhibit high therapeutic indices are preferred. The data obtained from these cell culture assays and animal studies can be used in formulating a range of dosage for use in humans. The dosage of such compounds lies preferably within a range of circulating concentrations that include the ED₅₀ with little or no toxicity. The dosage may vary within this range depending upon the dosage form employed and the route of administration utilized. The exact formulation, route of administration and dosage can be chosen by the individual physician in view of the patient's condition. (See e.g. Fingl *et al*., 1975, in "The Pharmacological Basis of Therapeutics", Ch. 1 p1). In the treatment of crises, the administration of an acute bolus or an infusion approaching the MTD may be required to obtain a rapid response.

Dosage amount and interval may be adjusted individually to provide plasma levels of the active moiety which are sufficient to maintain the kinase modulating effects, or minimal effective concentration (MEC). The MEC will vary for each compound but can be estimated from *in vitro* data; e.g. the concentration necessary to achieve 50-90% inhibition of protein kinase using the assays described herein. Dosages necessary to achieve the MEC will depend on individual characteristics and route of administration. However, HPLC assays or bioassays can be used to determine plasma concentrations.

Dosage intervals can also be determined using the MEC value. Compounds should be administered using a regimen which maintains plasma levels above the MEC for 10-90% of the time, preferably between 30-90% and most preferably between 50-90% until the desired amelioration of symptoms is achieved. In cases of local administration or selective uptake, the effective local concentration of the drug may not be related to plasma concentration.

The amount of composition administered will, of course, be dependent on the subject being treated, on the subject's weight, the severity of the affliction, the manner of administration and the judgment of the prescribing physician.

### Packaging

The compositions may, if desired, be presented in a pack or dispenser device which may contain one or more unit dosage forms containing the active ingredient. The pack may for example comprise metal or plastic foil, such as a blister pack. The pack or dispenser device may be accompanied by instructions for administration. Compositions comprising a compound of the invention formulated in a compatible pharmaceutical carrier may also be prepared, placed in an appropriate container, and labeled for treatment of an indicated condition.

In some formulations it may be beneficial to use the compounds of the present invention in the form of particles of very small size, for example as obtained by fluid energy milling.

In the compositions of the present invention the active compound may, if desired, be associated with other compatible pharmacologically active ingredients. For example, the compounds of this invention can be administered in combination with one or more additional pharmaceutical agents that inhibit or prevent the production of VEGF, attenuate intracellular responses to VEGF, block intracellular signal transduction, inhibit vascular hyperpermeability, reduce inflammation, or inhibit or prevent the formation of edema or neovascularization. The compounds of the invention can be administered prior to, subsequent to or simultaneously with the additional pharmaceutical agent, whichever course of administration is appropriate. The additional pharmaceutical agents include but are not limited to anti-edemic steroids, NSAIDS, ras inhibitors, anti-TNF agents, anti-ILl agents, antihistamines, PAF-antagonists, COX-1 inhibitors, COX-2 inhibitors, NO synthase inhibitors, PKC inhibitors and PI3 kinase inhibitors. The compounds of the invention and the additional pharmaceutical agents act either additively or synergistically. Thus, the administration of such a combination of substances that inhibit vascular hyperpermeability and/or inhibit the formation of edema can provide greater relief from the deletrious effects of a hyperproliferative disorder, angiogenesis, vascular hyperpermeability or edema than the administration of either substance alone. In the treatment of malignant disorders combinations with antiproliferative or cytotoxic chemotherapies or radiation are anticipated.

The present invention also comprises the use of a compound of formula I as a medicament.

Both the Src and Syk families of kinases play pivotal roles in the regulation of immune function. The Src family currently includes Fyn, Lck, Fgr, Fes, Lyn, Src, Yes, Hck, and Blk. The Syk family is currently understood to include only Zap and Syk. The Janus family of kinases is involved in the transduction of growth factor and proinflammatory cytokine signals through a number of receptors. Although BTK and ITK, members of the Tec family of kinases, play a less well understood role in immunobiology, their modulation by an inhibitor may prove therapeutically beneficial. The kinases RIP, IRAK-1, IRAK-2, NIK, IKK-1 and IKK-2 are involved in the signal transduction pathways for the key pro-inflammatory cytokines TNF and IL-1. By virtue of their ability to inhibit one or more of these kinases, compounds of formula I may function as immunomodulatory agents useful for the maintenance of allografts and the treatment of autoimmune disorders. Through their ability to regulate T cell activation or the potentiation of an inflammatory process, these compounds could be used to treat such autoimmune diseases. Transplants due to rejection phenomena, either host versus graft for solid organs or graft versus host for bone marrow, are limited by the toxicity of currently available immunosuppressive agents and would benefit from an efficacious drug with improved therapeutic index. Gene targeting experiments have demonstrated the essential role of Src in the biology of osteoclasts, the cells responsible for bone resorption. Compounds of formula I, through their ability to regulate Src, may also be useful in the treatment of osteoporosis, osteopetrosis, Paget's disease, tumor-induced hypercalcemia and in the treatment of bone metastases.

A number of protein kinases have been demonstrated to be protooncogenes. Chromosome breakage (at the ltk kinase break point on chromosome 5), translocation as in the case of the Abl gene with BCR (Philadelphia chromosome), truncation in instances such as c-Kit or EGFR, or mutation (e.g., Met) result in the creation of dysregulated proteins converting them from protooncogene to oncogene products. In other tumors, oncogenesis is driven by an autocrine or paracrine ligand/growth factor receptor interactions. Members of the src-family kinases are typically involved in downstream signal transduction thereby potentiating the oncogenesis and themselves may become oncogenic by over-expression or mutation. By inhibiting the protein kinase activity of these proteins the disease process may be disrupted. Vascular restenosis may involve process of FGF and/or PDGF - promoted smooth muscle and endothelial cell proliferation. Inhibition of FGFr or PDGFr kinase activity may be an efficacious strategy for inhibiting this phenomenon. Thus compounds of formula I which inhibit the kinase activity of normal or aberrant c-kit, c-met, c-fms, src-family members, EGFr, erbB2, erbB4, BCR-Ab1, PDGFr, FGFr, and other receptor or cytosolic tyrosine kinases may be of value in the treatment of benign and neoplastic proliferative diseases.

In many pathological conditions (for example, solid primary tumors and metastases, Kaposi's sarcoma, rheumatoid arthritis, blindness due to inappropriate ocular neovascularization, psoriasis and atherosclerosis) disease progression is contingent upon persistent angiogenesis. Polypeptide growth factors often produced by the disease tissue or associated inflammatory cells, and their corresponding endothelial cell specific receptor tyrosine kinases (e.g., KDR/VEGFR-2, Fit-1/VEGFR-1, Tie-2/Tek and Tie) are essential for the stimulation of endothelial cell growth, migration, organization, differentiation and the establishment of the requisite new functional vasculature.

As a result of the "vascular permeability factor" activity of VEGF in mediating vascular hyperpermeability, VEGF-stimulation of a VEGFR kinase is also believed to play an important role in the formation of tumor ascites, cerebral and pulmonary edema, pleural and pericardial effusions, delayed-type hypersensitivity reactions, tissue edema and organ dysfunction following trauma, bums, ischemia, diabetic complications, endometriosis, adult respiratory distress syndrome (ARDS), post-cardiopulmonary bypass-related hypotension and hyperpermeability, and ocular edema leading to glaucoma or blindness due to inappropriate neovascularization. In addition to VEGF, recently identified VEGF-C and VEGF-D, and HIV-Tat protein can also cause a vascular hyperpermeability response through the stimulation of a VEGFR kinase.

Tie-2 is expressed also in a select population of hematopoietic stem cells in which it may play a role in their recruitment, adhesion, regulation and differentiation (*Blood* **89**, 4317-4326 (1997)); this Tie-2 expressing population may serve as circulating angiogenic endothelial progenitors. Certain agents according to formula I capable of blocking the kinase activity of endothelial cell specific kinases could therefore inhibit disease progression involving these situations.

The compounds of formula I or a salt thereof or pharmaceutical compositions containing a therapeutically effective amount thereof may be used in the treatment of benign and neoplastic proliferative diseases and disorders of the immune system. Such diseases include autoimmune diseases, such as rheumatoid arthritis, thyroiditis, type 1 diabetes, multiple sclerosis, sarcoidosis, inflammatory bowel disease, myasthenia gravis and systemic lupus erythematosus; psoriasis, organ transplant rejection (eg. kidney rejection, graft versus host disease), benign and neoplastic proliferative diseases, human cancers such as lung, breast, stomach, bladder, colon, pancreas, ovarian, prostate and rectal cancer and hematopoietic cells (leukemia and lymphoma), and diseases involving inappropriate vascularization for example diabetic retinopathy, choroidal neovascularization due to age-related macular degeneration, and infantile hemangiomas in human beings. In addition, such inhibitors may be useful in the treatment of disorders involving VEGF mediated edema, ascites, effusions, and exudates, including for example macular edema, cerebral edema, and adult respiratory distress syndrome (ARDS).

The compounds of the present invention may also be useful in the prophylaxis of the above diseases.

A further aspect of the present invention provides the use of a compound of formula I or a salt thereof in the manufacture of a medicament for treating vascular hyperpermeability, angiogenesis-dependent disorders, proliferative diseases and/or disorders of the immune system in mammals, particularly human beings.

The present invention also provides a method of treating vascular hyperpermeability, inappropriate neovascularization, proliferative diseases and/or disorders of the immune system which comprises the administration of a therapeutically effective amount of a compound of formula I to a mammal, particularly a human being, in need thereof.

The in vitro potency of compounds in inhibiting these protein kinases may be determined by the procedures detailed below.

The potency of compounds can be determined by the amount of inhibition of the phosphorylation of an exogenous substrate (e.g., synthetic peptide (Z. Songyang et al., Nature. 373:536-539) by a test compound relative to control.

### KDR Tyrosine Kinase Production Using Baculovirus System:

The coding sequence for the human KDR intra-cellular domain (aa789-1354) was generated through PCR using cDNAs isolated from HUVEC cells. A poly-His6 sequence was introduced at the N-terminus of this protein as well. This fragment was cloned into transfection vector pVL1393 at the Xba 1 and Not 1 site. Recombinant baculovirus (BV) was generated through co-transfection using the BaculoGold Transfection reagent (PharMingen). Recombinant BV was plaque purified and verified through Western analysis. For protein production, SF-9 cells were grown in SF-900-II medium at 2 x 106/ml, and were infected at 0.5 plaque forming units per cell (MOI). Cells were harvested at 48 hours post infection.

### Purification of KDR

SF-9 cells expressing (His)₆KDR(aa789-1354) were lysed by adding 50 ml of Triton X-100 lysis buffer (20 mM Tris, pH 8.0, 137 mM NaCI, 10% glycerol, 1% Triton X-100, 1mM PMSF, 10µg/ml aprotinin, 1 µg/ml leupeptin) to the cell pellet from 1L of cell culture. The lysate was centrifuged at 19,000 rpm in a Sorval SS-34 rotor for 30 min at 4°C. The cell lysate was applied to a 5 ml NiCl₂ chelating sepharose column, equilibrated with 50 mM HEPES, pH7.5, 0.3 M NaCl. KDR was eluted using the same buffer containing 0.25 M imidazole. Column fractions were analyzed using SDS-PAGE and an ELISA assay (below) which measures kinase activity. The purified KDR was exchanged into 25mM HEPES, pH7.5, 25mM NaCl, 5 mM DTT buffer and stored at -80°C.

### Human Tie-2 Kinase Production and Purification

The coding sequence for the human Tie-2 intra-cellular domain (aa775-1124) was generated through PCR using cDNAs isolated from human placenta as a template. A poly-His₆ sequence was introduced at the N-terminus and this construct was cloned into transfection vector pVL 1939 at the Xba 1 and Not 1 site. Recombinant BV was generated through co-transfection using the BaculoGold Transfection reagent (PharMingen). Recombinant BV was plaque purified and verified through Western analysis. For protein production, SF-9 insect cells were grown in SF-900-II medium at 2 x 10⁶/ml, and were infected at MOI of 0.5. Purification of the His-tagged kinase used in screening was analogous to that described for KDR.

### Human Flt-1 Tyrosine Kinase Production and Purification

The baculoviral expression vector pVL1393 (Phar Mingen, Los Angeles, CA) was used. A nucleotide sequence encoding poly-His6 was placed 5' to the nucleotide region encoding the entire intracellular kinase domain of human Flt-1 (amino acids 786-1338). The nucleotide sequence encoding the kinase domain was generated through PCR using cDNA libraries isolated from HUVEC cells. The histidine residues enabled affinity purification of the protein as a manner analogous to that for KDR and ZAP70. SF-9 insect cells were infected at a 0.5 multiplicity and harvested 48 hours post infection.

### EGFR Tyrosine Kinase Source

EGFR was purchased from Sigma (Cat # E-3641; 500 units/50 µl) and the EGF ligand was acquired from Oncogene Research Products/Calbiochem (Cat # PF011-100).

### Expression of ZAP70

The baculoviral expression vector used was pVL1393. (Pharmingen, Los Angeles, Ca.) The nucleotide sequence encoding amino acids M(H)6 LVPR₉S was placed 5' to the region encoding the entirety of ZAP70 (amino acids 1-619). The nucleotide sequence encoding the ZAP70 coding region was generated through PCR using cDNA libraries isolated from Jurkat immortalized T-cells. The histidine residues enabled affinity purification of the protein (vide infra). The LVPR₉S bridge constitutes a recognition sequence for proteolytic cleavage by thrombin, enabling removal of the affinity tag from the enzyme. SF-9 insect cells were infected at a multiplicity of infection of 0.5 and harvested 48 hours post infection.

### Extraction and purification of ZAP70

SF-9 cells were lysed in a buffer consisting of 20 mM Tris, pH 8.0, 137 mM NaCl, 10% glycerol, 1% Triton X-100, 1 mM PMSF, 1 µg/ml leupeptin, 10 µg/ml aprotinin and 1 mM sodium orthovanadate. The soluble lysate was applied to a chelating sepharose HiTrap column (Pharmacia) equilibrated in 50 mM HEPES, pH 7.5, 0.3 M NaCl. Fusion protein was eluted with 250 mM imidazole. The enzyme was stored in buffer containing 50 mM HEPES, pH 7.5, 50 mM NaCl and 5 mM DTT.

### Lck source

Lck or truncated forms of Lck may be commercially obtained (e.g. from Upstate Biotechnology Inc. (Saranac Lake, N.Y) and Santa Cruz Biotechnology Inc. (Santa Cruz, Ca.)) or purified from known natural or recombinant sources using conventional methods.

### Cdc2 source

The human recombinant enzyme and assay buffer may be obtained commercially (New England Biolabs, Beverly, MA. USA) or purified from known natural or recombinant sources using conventional methods.

### Cdc2 Assay

The protocol used was that provided with the purchased reagents with minor modifications. In brief, the reaction was carried out in a buffer consisting of 50mM Tris pH 7.5, 100mM NaCl, 1mM EGTA, 2mM DTT, 0.01% Brij, 5% DMSO and 10mM MgCl₂ (commercial buffer) supplemented with fresh 300 µM ATP (31 µCi/ml) and 30 µg/ml histone type IIIss final concentrations. A reaction volume of 80µL, containing units of enzyme, was run for 20 minutes at 25 degrees C in the presence or absence of inhibitor. The reaction was terminated by the addition of 120µL of 10% acetic acid. The substrate was separated from unincorporated label by spotting the mixture on phosphocellulose paper, followed by 3 washes of 5 minutes each with 75mM phosphoric acid. Counts were measured by a betacounter in the presence of liquid scintillant.
Certain compounds of this invention significantly inhibit cdc2 at concentrations below 50 µM.

### PKC kinase source

The catalytic subunit of PKC may be obtained commercially (Calbiochem).

### PKC kinase assay

A radioactive kinase assay was employed following a published procedure (Yasuda, I., Kirshimoto, A., Tanaka, S., Tominaga, M., Sakurai, A., Nishizuka, Y. *Biochemical and Biophysical Research Communication 3*:166, 1220-1227 (1990)). Briefly, all reactions were performed in a kinase buffer consisting of 50 mM Tris-HCl pH7.5, 10mM MgCl₂, 2mM DTT, 1mM EGTA, 100 µM ATP, 8 µM peptide, 5% DMSO and ³³P ATP (8Ci/mM). Compound and enzyme were mixed in the reaction vessel and the reaction initiated by addition of the ATP and substrate mixture. Following termination of the reaction by the addition of 10 µL stop buffer (5 mM ATP in 75mM phosphoric acid), a portion of the mixture was spotted on phosphocellulose filters. The spotted samples were washed 3 times in 75 mM phosphoric acid at room temperature for 5 to 15 minutes. Incorporation of radiolabel was quantified by liquid scintillation counting.

### Erk2 enzyme source

The recombinant murine enzyme and assay buffer may be obtained commercially (New England Biolabs, Beverly MA. USA) or purified from known natural or recombinant sources using conventional methods.

### Erk2 enzyme assay

In brief, the reaction was carried out in a buffer consisting of 50 mM Tris pH 7.5, 1mM EGTA, 2mM DTT, 0.01% Brij, 5% DMSO and 10 mM MgCl₂ (commercial buffer) supplemented with fresh 100 µM ATP (31 µCi/ml) and 30µM myelin basic protein under conditions recommended by the supplier. Reaction volumes and method of assaying incorporated radioactivity were as described for the PKC assay (vide supra).

### Enzyme Linked Immunosorbent Assay (ELISA) For PTKs

Enzyme linked immunosorbent assays (ELISA) were used to detect and measure the presence of tyrosine kinase activity. The ELISA were conducted according to known protocols which are described in, for example, Voller, *et al*., 1980, "Enzyme-Linked Immunosorbent Assay," In: *Manual of Clinical Immunology, 2d ed*., edited by Rose and Friedman, pp 359-371 Am. Soc. of Microbiology, Washington, D.C.

The disclosed protocol was adapted for determining activity with respect to a specific PTK. For example, preferred protocols for conducting the ELISA experiments is provided below. Adaptation of these protocols for determining a compound's activity for other members of the receptor PTK family, as well as non-receptor tyrosine kinases, are well within the abilities of those in the art. For purposes of determining inhibitor selectivity, a universal PTK substrate (e.g., random copolymer of poly(Glu₄ Tyr), 20,000-50,000 MW) was employed together with ATP (typically 5 µM) at concentrations approximately twice the apparent Km in the assay.

The following procedure was used to assay the inhibitory effect of compounds of this invention on KDR, Flt-1, Tie-2, EGFR and ZAP70 tyrosine kinase activity:

### Buffers and Solutions:

**PGT:** Poly (Glu,Tyr) 4:1
   Store powder at -20°C. Dissolve powder in phosphate buffered saline (PBS) for 50mg/ml solution. Store 1ml aliquots at -20°C. When making plates dilute to 250µg/ml in Gibco PBS.
**Reaction Buffer:** 100mM Hepes, 20mM MgCl₂, 4mM MnCl₂, 5mM DTT, 0.02%BSA, 200µM NaVO₄, pH 7.10
**ATP:** Store aliquots of 100mM at -20°C. Dilute to 20uM in water
Washing Buffer: PBS with 0.1% Tween 20
**Antibody Diluting Buffer:** 0.1% bovine serum albumin (BSA) in PBS
**TMB Substrate:** mix TMB substrate and Peroxide solutions 9:1 just before use or
   use K-Blue Substrate from Neogen
**Stop Solution:** 1M Phosphoric Acid

### Procedure

### 1. Plate Preparation:

Dilute PGT stock (50mg/ml, frozen) in PBS to a 250µg/ml. Add 125µl per well of Corning modified flat bottom high affinity ELISA plates (Coming #25805-96). Add 125µl PBS to blank wells. Cover with sealing tape and incubate overnight 37°C.
Wash 1x with 250µl washing buffer and dry for about 2hrs in 37°C dry incubator. Store coated plates in sealed bag at 4°C until used.

### 2. Tyrosine Kinase Reaction:

Prepare inhibitor solutions at a 4x concentration in 20% DMSO in water. Prepare reaction buffer
Prepare enzyme solution so that desired units are in 50µl, e.g. for KDR make to 1 ng/µl for a total of 50ng per well in the reactions. Store on ice.
Make 4x ATP solution to 20µM from 100mM stock in water. Store on ice
Add 50µl of the enzyme solution per well (typically 5-50 ng enzyme/well depending on the specific activity of the kinase)
Add 25µl 4x inhibitor
Add 25µl 4x ATP for inhibitor assay
Incubate for 10 minutes at room temperature
Stop reaction by adding 50µl 0.05N HCl per well
Wash plate

### **Final Concentrations for Reaction: 5µM ATP, 5% DMSO

### 3. Antibody Binding

Dilute 1mg/ml aliquot of PY20-HRP (Pierce) antibody (a phosphotyrosine antibody) to 50ng/ml in 0.1% BSA in PBS by a 2 step dilution (100x, then 200x)
Add 100µl Ab per well. Incubate 1 hr at room temp. Incubate lhr at 4C.
Wash 4x plate

### 4. Color reaction

Prepare TMB substrate and add 100µl per well
Monitor OD at 650nm until 0.6 is reached
Stop with 1M Phosphoric acid. Shake on plate reader.
Read OD immediately at 450nm

Optimal incubation times and enzyme reaction conditions vary slightly with enzyme preparations and are determined empirically for each lot.
For Lck, the Reaction Buffer utilized was 100 mM MOPSO, pH 6.5, 4 mM MnCl₂, 20 mM MgCl₂, 5 mM DTT, 0.2% BSA, 200 mM NaVO₄ under the analogous assay conditions.

Compounds of formula I can have therapeutic utility in the treatment of diseases involving both identified, including those not mentioned herein, and as yet unidentified protein tyrosine kinases which are inhibited by compounds of formula I. All compounds exemplified herein significantly inhibit KDR kinase at concentrations of 50 micromolar or below, Some compounds of this invention also significantly inhibit other PTKs such as 1ck at concentrations of 50 micromolar or below.

### In Vitro Models for T-cell Activation

Upon activation by mitogen or antigen, T-cells are induced to secrete IL-2, a growth factor that supports their subsequent proliferative phase. Therefore, one may measure either production of IL-2 from or cell proliferation of, primary T-cells or appropriate T-cell lines as a surrogate for T-cell activation. Both of these assays are well described in the literature and their parameters well documented (in Current Protocols in Immunology, Vol 2, 7.10.1-7.11.2).

In brief, T-cells may be activated by co-culture with allogenic stimulator cells, a process termed the one-way mixed lymphophocyte reaction. Responder and stimulator peripheral blood mononuclear cells are purified by Ficoll-Hypaque gradient (Pharmacia) per directions of the manufacturer. Stimulator cells are mitotically inactivated by treatment with mitomycin C (Sigma) or gamma irradiation. Responder and stimulator cells are co-cultured at a ratio of two to one in the presence or absence of the test compound. Typically 10⁵ responders are mixed with 5 x 10⁴ stimulators and plated (200 µl volume) in a U bottom microtiter plate (Costar Scientific). The cells are cultured in RPMI 1640 supplemented with either heat inactivated fetal bovine serum (Hyclone Laboratories), or pooled human AB serum from male donors, 5 x 10⁻⁵ M 2mercaptoethanol and 0.5% DMSO, The cultures are pulsed with 0.5 µCi of ³H thymidine (Amersham) one day prior to harvest (typically day three). The cultures are harvested (Betaplate harvester, Wallac) and isotope uptake assessed by liquid scintillation (Betaplate, Wallac).

The same culture system may be used for assessing T-cell activation by measurement of IL-2 production. Eighteen to twenty-four hours after culture initiation, the supematants are removed and the IL-2 concentration is measured by ELISA (R and D Systems) following the directions of the manufacturer.

### In-vivo Models of T-Cell Activation

The *in vivo* efficacy of compounds can be tested in animal models known to directly measure T-cell activation or for which T-cells have been proven the effectors. T-cells can be activated in *vivo* by ligation of the constant portion of the T-cell receptor with a monoclonal anti-CD3 antibody (Ab). In this model, BALB/c mice are given 10µg of anti-CD3 Ab intraperitoneally two hours prior to exsanguination. Animals to receive a test drug are pre-treated with a single dose of the compound one hour prior to anti-CD3 Ab administration. Serum levels of the proinflammatory cytokines interferon-y (IFN- γ) and tumor necrosis factor-α(TNF-α), indicators of T-cell activation, are measured by ELISA. A similar model employs *in vivo* T-cell priming with a specific antigen such as keyhole limpet hemocyanin (KLH) followed by a secondary *in vitro* challenge of draining lymph node cells with the same antigen. As previously, measurement of cytokine production is used to assess the activation state of the cultured cells. Briefly, C57BL/6 mice are immunized subcutaneously with 100 µg KLH emulsified in complete Freund's adjuvant (CFA) on day zero. Animals are pre-treated with the compound one day prior to immunization and subsequently on days one, two and three post immunization. Draining lymph nodes are harvested on day 4 and their cells cultured at 6 x 10⁶ per ml in tissue culture medium (RPMI 1640 supplemented with heat inactivated fetal bovine serum (Hyclone Laboratories), 5 x 10⁻⁵ M 2-mercaptoethanol and 0.5% DMSO) for both twenty-four and forty-eight hours. Culture supernatants are then assessed for the autocrine T-cell growth factor Interleukin-2 (IL-2) and/or IFN-γ levels by ELISA.

Lead compounds can also be tested in animal models of human disease. These are exemplified by experimental auto-immune encephalomyelitis (EAE) and collagen-induced arthritis (CIA). EAE models which mimic aspects of human multiple sclerosis have been described in both rats and mice (reviewed FASEB J. 5:2560-2566, 1991; murine model: Lab. Invest. 4(3):278, 1981; rodent model:J. Immunol 146(4):1163-8, 1991 ). Briefly, mice or rats are immunized with an emulsion of myelin basic protein (MBP), or neurogenic peptide derivatives thereof, and CFA. Acute disease can be induced with the addition of bacterial toxins such as *bordetella pertussis*. Relapsing/remitting disease is induced by adoptive transfer of T-cells from MBP/ peptide immunized animals.

CIA may be induced in DBA/1 mice by immunization with type II collagen (J. Immunol:142(7):2237-2243). Mice will develop signs of arthritis as early as ten days following antigen challenge and may be scored for as long as ninety days after immunization. In both the EAE and CIA models, a compound may be administered either prophylactically or at the time of disease onset. Efficacious drugs should reduce severity and/or incidence.

Certain compounds of this invention which inhibit one or more angiogenic receptor PTK, and/or a protein kinase such as 1ck involved in mediating inflammatory responses can reduce the severity and incidence of arthritis in these models.

Compounds can also be tested in mouse allograft models, either skin (reviewed in Ann. Rev. Immunol., 10:333-58, 1992; Transplantation: 57(12): 1701-17D6, 1994) or heart (Am.J.Anat.:113:273, 1963). Briefly, full thickness skin grafts are transplanted from C57BL/6 mice to BALB/c mice. The grafts are examined daily, beginning at day six, for evidence of rejection. In the mouse neonatal heart transplant model, neonatal hearts are ectopically transplanted from C57BL/6 mice into the ear pinnae of adult CBA/J mice. Hearts start to beat four to seven days post transplantation and rejection may be assessed visually using a dissecting microscope to look for cessation of beating.

### Cellular Receptor PTK Assays

The following cellular assay was used to determine the level of activity and effect of the different compounds of the present invention on KDR/VEGFR2. Similar receptor PTK assays employing a specific ligand stimulus can be designed along the same lines for other tyrosine kinases using techniques well known in the art.

VEGF-Induced KDR Phosphorylation in Human Umbilical Vein Endothelial Cells (HUVEC) as Measured by Western Blots.
1. HUVEC cells (from pooled donors) were purchased from Clonetics (San Diego, CA) and cultured according to the manufacturer directions. Only early passages (3-8) were used for this assay. Cells were cultured in 100 mm dishes (Falcon for tissue culture; Becton Dickinson; Plymouth, England) using complete EBM media (Clonetics).
2. For evaluating a compound's inhibitory activity, cells were trypsinized and seeded at 0.5-1.0 x 10⁵ cells/well in each well of 6-well cluster plates (Costar; Cambridge, MA).
3. 3-4 days after seeding, plates were 90-100% confluent. Medium was removed from all the wells, cells were rinsed with 5-10ml of PBS and incubated 18-24h with 5ml of EBM base media with no supplements added (i.e., serum starvation).
4. Serial dilutions of inhibitors were added in 1ml of EBM media (25µM, 5µM, or 1µM final concentration to cells and incubated for one hour at 37°C. Human recombinant VEGF₁₆₅ ( R & D Systems) was then added to all the wells in 2 ml of EBM medium at a final concentration of 50ng/ml and incubated at 37°C for 10 minutes. Control cells untreated or treated with VEGF only were used to assess background phosphorylation and phosphorylation induction by VEGF.

All wells were then rinsed with 5-10ml of cold PBS containing 1mM Sodium Orthovanadate (Sigma) and cells were lysed and scraped in 200µl of RIPA buffer (50mM Tris-HCl) pH7, 150mM NaCI, 1% NP-40, 0.25% sodium deoxycholate, 1mM EDTA) containing protease inhibitors (PMSF 1 mM, aprotinin 1µg/ml, pepstatin 1µg/ml, leupeptin 1µg/ml, Na vanadate 1mM, Na fluoride 1mM) and 1µg/ml of Dnase (all chemicals from Sigma Chemical Company, St Louis, MO). The lysate was spun at 14,000 rpm for 30min, to eliminate nuclei.

Equal amounts of proteins were then precipitated by addition of cold (-20°C) Ethanol (2 volumes) for a minimum of 1 hour or a maximum of overnight. Pellets were reconstituted in Laemli sample buffer containing 5% β-mercaptoethanol (BioRad; Hercules, CA) and boiled for 5min. The proteins were resolved by polyacrylamide gel electrophoresis (6%, 1.5mm Novex, San Deigo, CA) and transferred onto a nitrocellulose membrane using the Novex system. After blocking with bovine serum albumin (3%), the proteins were probed overnight with anti-KDR polyclonal antibody (C20, Santa Cruz Biotechnology; Santa Cruz, CA) or with anti-phosphotyrosine monoclonal antibody (4G10, Upstate Biotechnology, Lake Placid, NY) at 4°C. After washing and incubating for 1 hour with HRP-conjugated F(ab)₂ of goat anti-rabbit or goat-anti-mouse IgG the bands were visualized using the emission chemiluminescience (ECL) system (Amersham Life Sciences, Arlington Height, IL).

Certain examples of the present invention significantly inhibit cellular VEGF-induced KDR tyrosine kinase phosphorylation at concentrations of less than 50 µM.

### In Vivo Uterine Edema Model

This assay measures the capacity of compounds to inhibit the acute increase in uterine weight in mice which occurs in the first few hours following estrogen stimulation. This early onset of uterine weight increase is known to be due to edema caused by increased permeability of uterine vasculature. Cullinan-Bove and Koss (*Endocrinology* (1993), *133*:829-837) demonstrated a close temporal relationship of estrogen-stimulated uterine edema with increased expression of VEGF mRNA in the uterus. These results have been confirmed by the use of neutralizing monoclonal antibody to VEGF which significantly reduced the acute increase in uterine weight following estrogen stimulation (WO 97/42187). Hence, this system can serve as a model for *in vivo* inhibition of VEGF signalling and the associated hyperpermeability and edema.
Materials: All hormones were purchased from Sigma (St. Louis, MO) or Cal Biochem (La Jolla, CA) as lyophilized powders and prepared according to supplier instructions.
Vehicle components (DMSO, Cremaphor EL) were purchased from Sigma (St. Louis, MO).
Mice (Balb/c, 8-12 weeks old) were purchased from Taconic (Germantown, NY) and housed in a pathogen-free animal facility in accordance with institutional Animal Care and Use Committee Guidelines.

### Method:

- Day 1:: Balb/c mice were given an intraperitoneal (i.p.) injection of 12.5 units of pregnant mare's serum gonadotropin (PMSG).
- Day 3:: Mice received 15 units of human chorionic gonadotropin (hCG) i.p.
- Day 4:: Mice were randomized and divided into groups of 5-10. Test compounds were administered by i.p., i.v. or p.o. routes depending on solubility and vehicle at doses ranging from 1-100 mg/kg. Vehicle control group received vehicle only and two groups were left untreated.

Thirty minutes later, experimental, vehicle and 1 of the untreated groups were given an i.p. injection of 17 β-estradiol (500 µg/kg). After 2-3 hours, the animals were sacrificed by CO₂ inhalation. Following a midline incision, each uterus was isolated and removed by cutting just below the cervix and at the junctions of the uterus and oviducts. Fat and connective tissue were removed with care not to disturb the integrity of the uterus prior to weighing. Mean weights of treated groups were compared to untreated or vehicle treated groups. Significance was determined by Student's t-test. Non-stimulated control group was used to monitor estradiol response.

Results demonstrate that certain compounds of the present invention inhibit the formation of edema when administered systemically by various routes.

Certain compounds of this invention which are inhibitors of angiogenic receptor tyrosine kinases can also be shown active in a Matrigel implant model of neovascularization. The Matrigel neovascularization model involves the formation of new blood vessels within a clear "marble" of extracellular matrix implanted subcutaneously which is induced by the presence of proangiogenic factor producing tumor cells (for examples see: Passaniti, A., et al, Lab. Investig. (1992), 67(4), 519-528; Anat. Rec. (1997), 249(1), 63-73; Int. J. Cancer (1995), 63(5), 694-701; Vasc. Biol. (1995), 15(11), 1857-6). The model preferably runs over 3-4days and endpoints include macroscopic visual/image scoring of neovascularization, microscopic microvessel density determinations, and hemoglobin quantitation (Drabkin method) following removal of the implant versus controls from animals untreated with inhibitors. The model may alternatively employ bFGF or HGF as the stimulus.

Certain compounds of this invention which inhibit one or more oncogenic, protooncogenic, or proliferation-dependent protein kinases, or angiogenic receptor PTK also inhibit the growth, of primary murine, rat or human xenograft tumors in mice, or inhibit metastasis in murine models.

### EXEMPLIFICATIONS

### I. Synthesis

The compounds of formula I may be prepared as described below. In the following

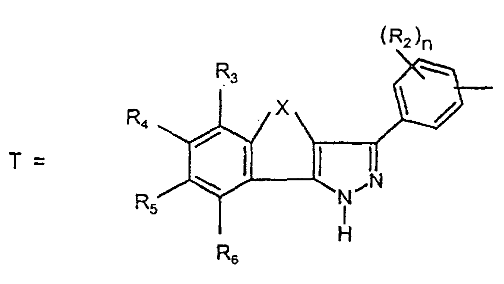

Compounds of formula I in which A represents CONH may be prepared by reacting a compound of formula TL₁ CORₜ in which Rₜ is a leaving group for example halo or alkoxy with an amine of formula H₂N-L₂-R₁ at a temperature in the range of 0-250°C optionally in the presence of a solvent.

Compounds of formula I in which L₁A represents CH₂NH may be prepared by reacting a compound of formula I in which A represents CONH with a reducing agent, for example lithium aluminium hydride at a temperature in the range of 0-250°C optionally in the presence of a solvent.

Alternatively, compounds of formula I in which L₁A represents CH₂NH may be prepared by reacting a compound of formula TL₁ CHO with an amine of formula H₂N-L₂-R₁ in the presence of a reducing agent, for example sodium triacetoxyborohydride, at a temperature in the range of 0-250°C optionally in the presence of a solvent.

Alternatively the group L₁-A-L₂-R₁ may be present in the phenyl ring and the ring system may be constructed as described below in which

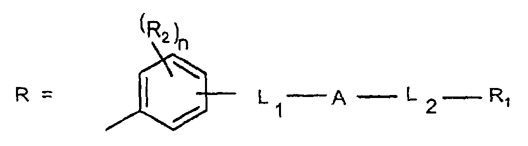

There are two general approaches to the synthesis of the ring systems of the compounds of formula I that have been set forth in U.S. Patent 3,843,665 and U.S. Patent 3,843,666.

In U.S. Patent 3,843,665, cyclization of the pyrazole ring is effected by heating compounds of formula II with an aromatic sulfonylhydrazide of formula III in an inert solvent and a catalytic amount of an acid. The reaction is carried out for a period of 5 to 30 hours preferably at a temperature of 75°C to 100°C and gives compounds of formula I in which R₁ is hydrogen, 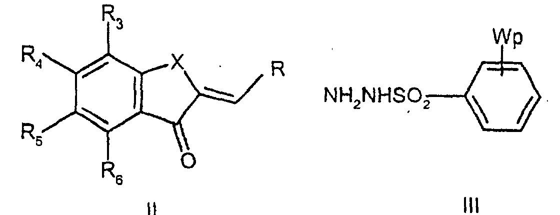 wherein:
p is 0, 1, 2; W is a lower alkyl ; and R, R₃, R₄, R₅, R₆ and X are as previously defined. Compounds of formula II are prepared by treating an appropriately
functionalized compound of formula IV with an aldehyde of formula V in the presence of an acid or base catalyst (Braun, R. A.; Mosher, W. A. J. Amer. Chem. Soc. 1958, 80, 2749). 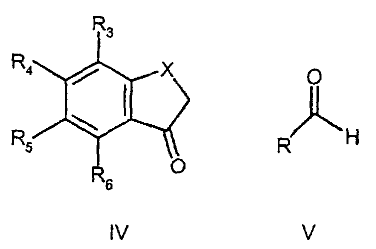 A second method of preparing the ring systems of the compounds of formula I is set forth by U.S. Patent 3,843,666 where cpmpounds with the general formula VI are heated to 75° to 175°C with a catalytic amount of an organic carboxylic acid or an organic sulfonic acid in an inert solvent such as an aromatic hydrocarbon for a period of 6 to 24 hours, 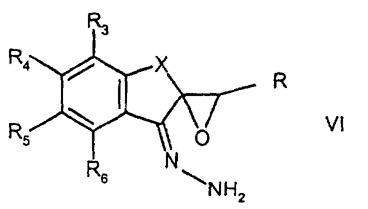
wherein: R, R₃, R₄, R₅, R₆ and X are as previously defined.

Compounds of formula VI are prepared by treating compounds of the general formula VII with hydrazine in an inert solvent. The reaction is carried out at 15° to 20°C for a period of up to 24 hours.

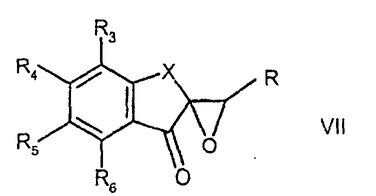

Alternatively compounds of formula I may be prepared directly by reacting a compound of formula VII with hydrazine without isolating the compound of formula VI, for example by heating a compound of formula VII with hydrazine in an inert solvent, e.g. methanol, in the presence of an acid catalyst, e.g. acetic acid, at a temperature in the range from 60°C to the boiling point of the inert solvent employed.

Compounds of formula I may also be prepared by reacting a compound of formula XVI 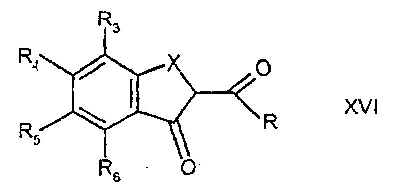 wherein:
R, R₃, R₄, R₅, R₆ and X are as previously defined with hydrazine in an inert solvent e.g. methanol, at a temperature in the range of from 15°C to the boiling point of the inert solvent employed.

Compounds which have the general formula VII are prepared by treating a compound of formula VIII with an aldehyde of formula V under basic conditions. The reaction is carried out in an inert solvent at a temperature between 5°C and 10°C for a period of 3 to 6 hours, 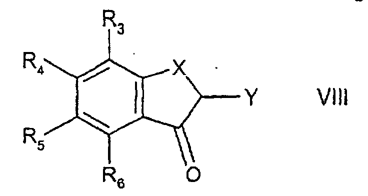 wherein:
Y is any conventional leaving group, such as chlorine, bromine, iodine, tosylate or mesylate and R₃, R₄, R₅, R₆ and X are as previously defined .

Compounds of formula VII may also be prepared by reacting a compound of formula II with an epoxidizing agent, for example hydrogen peroxide, in an inert solvent, for example methanol, dichloromethane, water or mixtures thereof, at a temperature in the range of 0° to 100°C optionally in the presence of a base, for example sodium hydroxide.

Cyclization of VI can also be effected by treatment with a mineral acid such as hydrochloric acid, sulfuric acid or phosphoric acid. The reaction is carried out in a lower alkanol at a temperature between 15°C to 20°C for a period of 12 to 48 hours. The product of the reaction, IX, can then be aromatized to I by heating to a temperature of 50°C to 150°C with an organic carboxylic acid or an organic sulfonic acid in a straight chain ether or cyclic ether for the period of 8 to 30 hours.

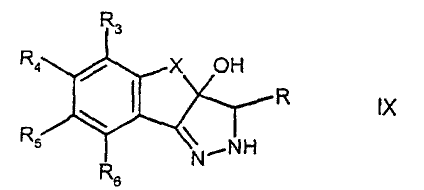

Compound IX can be diacetylated by treatment with an acid anhydride of formula (RₓCO)₂O (structure X) in which Rₓ is a C₁₋₄ alkyl group in an inert solvent such as an aromatic hydrocarbon at a temperature between 35°C to 200°C for a period of 5 to 8 hours to give a compound of formula XI.

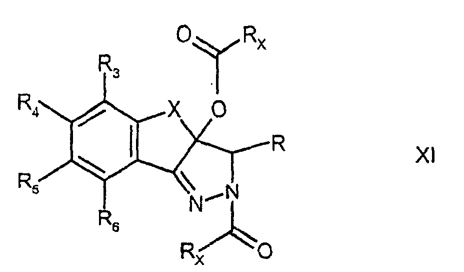

Compound XI can then be aromatized to compound XII by heating to a temperature of 35°C to 200°C with a mineral acid or an organic acid in an inert solvent for the period of 4 to 8 hours. Finally, compound XII can be converted to I by heating to a temperature of 50°C to 150°C in an inert solvent such as water or a lower alcohol in the presence of an alkali metal or an alkali metal hydroxide for the period of 8 to 30 hours.

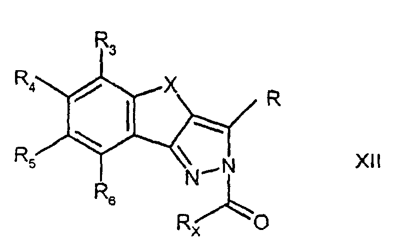

Compounds with the general formula II can be cyclized to compounds of formula XIII by reaction with hydrazine in an inert solvent, for example methanol, at a temperature in the range of 35-150°C.

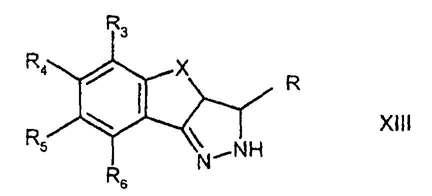

Compounds of formula I may be prepared by reacting a compound of formula XIII with a dehydrogenating agent, for example sulphur, oxygen, palladium, manganese dioxide or lead dioxide optionally in the presence of an inert solvent, for example a hydrocarbon, at a temperature in the range of 15 to 250°C.

Specific examples of the above transformations can be found in U.S. Patents 3,843,665 and 3,843,666.

The bridging carbonyl can be transformed to a methylene group via a Wolf-Kishner reduction of the corresponding hydrazone (Mosher, W.A., Tawfik, E.-Z., Lipp, D. W. J. Org. Chem. 1971, 36, 3890).

Additional methods for functionalization of the bridging carbonyl and specific examples can be found in Japanese Patent Application JP 60 130521 A2, and B. Loev, U.S. Patent 3,004,983 (1960).

Compounds of formula I may be prepared by reacting a compound of formula XIV 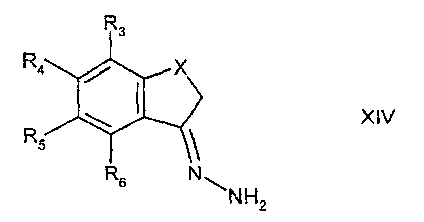 with a strong base, for example n-butyllithium at a temperature in the range of -78°C to 25°C, followed by reaction with a compound of formula R₂COG (structure XV) in which R₂ is as previously defined and G represents a C₁₋₆ alkoxy group.

Compounds of formula IV, VIII, XIV, XV and XVI are commercially available or may be prepared by methods known to those skilled in the art.

Compounds of formula I in which X represents SO, or SO₂ may be prepared by oxidizing a compound of formula I in which X represents S by methods known to those skilled in the art for example by using an appropriate number of molar equivalents of 3-chloroperbenzoic acid.

Compounds of formula I in which X represents a group of formula -C=NOR₇ may be prepared by reacting a compound of formula I in which X represents carbonyl, with a compound of formula H₂NOR₇ by methods known to those skilled in the art.

Compounds of formula I in which R₁ = 4-pyridyl may be further functionalized in the 2-position of the pyridine ring by methods known to those skilled in the art, for example, via pyridine-N-oxide mediated rearrangements.

Certain substituents in compounds of formula I may be interconverted by methods known to those skilled in the art. For example alkoxy substituents may be reacted with a suitable ether cleaving reagent for example hydrobromic acid, boron tribromide or pyridine hydrochloride to give a compound of formula I with a hydroxy substituent. Alternatively compounds of formula I with an alkoxy substituent may be prepared by alkylating compounds of formula I which have a hydroxy substituent. Carboxylic ester substituents may be converted into carboxy or amide substituents and carboxylic acid substituents may be converted into carboxylic ester or amide substituents. Nitro substituents may be reduced to amines and amines may be acylated by methods known to those skilled in the art.

It will be appreciated by those skilled in the art that certain substituents may react with some of the reagents described in the above processes. In such cases an alternative process should be used or the reactive substituent should be protected prior to the reaction and deprotected after the reaction.

The invention is illustrated by the following Examples which are given by way of example only. The final product of each of these Examples was characterized by one or more of the following procedures: high performance liquid chromatography; elemental analysis, nuclear magnetic resonance spectroscopy, infrared spectroscopy and high resolution mass spectroscopy. The following abbreviations are used
IMS = industrial methylated spirit
LCMS = liquid chromatography/mass spectroscopy.

### Example 1

a) A mixture of indan-1-one (3.3 g), methyl 4-formylbenzoate (5.0 g), piperidine (0.6 ml) and glacial acetic acid (0.5 ml) was heated on a steam bath for 3 hours. The solid mass obtained was boiled up in industrial methylated spirits (200 ml) and then hot filtered. The solid residue obtained was washed with industrial methylated spirits and dried to give methyl 4-(1-oxoindan-2-ylidenemethyl)benzoate, m.p. 194-198°C.
b) The product from a) (1.5 g) was suspended in methanol (10 ml) and dichloromethane (15 ml) and stirred at 0-5°C whilst 2M sodium hydroxide solution (2.7 ml) was added followed by 30% hydrogen peroxide (100 vol. 1.1 ml). The mixture was stirred at 0-5°C for 5 minutes then at ambient temperature for 24 hours. Dichloromethane (100 ml) was added to the mixture which was then washed with brine (2 x 50 ml), dried, filtered and evaporated to give methyl 4-(1-oxospiro[indan-2,2'-oxiran]-3'-yl)benzoate, m.p. 160-163°C. The aqueous phase was acidified with 5M hydrochloric acid and extracted with dichloromethane to give 4-(1-oxospiro[indan-2,2'-oxiran]-3'-yl)benzoic acid, m.p. 220°C with decomposition
c) 4-(1-Oxospiro[indan-2,2'-oxiran]-3'-yl)benzoic acid from part b) (780 mg), methanol (50 ml), hydrazine hydrate (0.18 ml) and glacial acetic acid (6 drops) were boiled under reflux for 24 hours. The mixture was cooled in ice and filtered to give 4-(1,4-dihydroindeno[1,2-c]pyrazol-3-yl)benzoic acid, m.p. >320°C.
d) A mixture of the product from c) (5.3 g) and dichloromethane (250 ml) was stirred at ambient temperature and then oxalyl chloride (5 ml) and dry N,N-dimethylformamide (6 drops) were added, the mixture was stirred at ambient temperature for 10 minutes and then boiled under reflux for 90 minutes. The solvent was removed under reduced pressure to give a crude acid chloride which was used directly in the next experiment.
e) The acid chloride from d) (3.73 g) was stirred at ambient temperature in dichloromethane (150 ml), then triethylamine (3 ml) and then 4-aminopyridine (0.9 g) were added. The mixture was stirred at ambient temperature for 3 hours. Water (150 ml) and 5M sodium hydroxide solution (50 ml) were added and the mixture was stirred for 90 minutes at ambient temperature. The mixture was filtered and the residue was washed with dichloromethane and water. The filtrate and washings were combined, separated and the dichloromethane layer was dried and evaporated to give a solid which was combined with the original solid obtained from the filtration and separated by flash column chromatography on silica using dichloromethane/ethyl alcohol (10:1) to give *N*-(4-pyridyl)-4-(1,4-dihydroindeno[1,2-c)pyrazol-3-yl)-benzamide.
f) Lithium aluminium hydride ( 830 mg) was added in portions to a stirred mixture of the product from e) (1.9 g) in dry tetrahydrofuran (50 ml) at ambient temperature. The mixture was boiled under reflux for 1 hour. The mixture was cooled to 0-5°C and added to ethyl acetate (70 ml) and then water (70 ml) was added. The mixture was stirred for 10 minutes and filtered to give a solid A. The aqueous layer was separated off and extracted with further ethyl acetate (100 ml). The solid A was stirred with ethanol and filtered. The ethyl acetate extracts and the ethanol filtrate were combined and evaporated. The residue obtained was purified by flash column chromatography using dichloromethane/ethanol (10:1) to give 4-(1,4-dihydroindeno[1,2-c]pyrazole)-N-(4-pyridyl)benzylamine, m.p. 270-274°C.

### Example 2

a) A mixture of indan-1-one (20.0 g), 4-nitrobenzaldehyde (27.0 g), glacial acetic acid (3.0 g) and piperidine (3.06 g) was heated at 95°C under nitrogen for 3.5 h. The mixture was cooled to 20°C and filtered to give a solid which was recrystallized from industrial methylated spirit to give 2-(4-nitrobenzylidene)indan-1-one.
b) The product from a) (28.0 g) was stirred with dichloromethane (100 ml) and methanol (100 ml) at 20°C and then 2M sodium hydroxide solution (50 ml) was added followed by hydrogen peroxide (20 ml, 100 volumes). The mixture was stirred at 20°C for 24 hours. Further hydrogen peroxide (10.0 ml, 100 volumes) was added and the mixture was stirred for a further 24 hours. Further hydrogen peroxide (10 ml, 100 volumes) was added and the mixture was stirred for 64 hours. The reaction mixture was neutralized with glacial acetic acid and the solid which formed was collected by filtration and dried to give 3'-(4-nitrophenyl)-1-oxospiro[indan-2,2'-oxirane].
c) The product from b) (10.0 g) was dissolved in ethanol (180 ml) and hydrazine hydrate (1.78 g) was added to the solution obtained, followed by glacial acetic acid (30 drops). The mixture was boiled under reflux for 5 hours and then cooled to 20°C and stood at this temperature for 18 hours. The solid was collected by filtration and recrystallized from acetone to give 3-(4-nitrophenyl)-1,4-dihydroindeno[1,2-c]pyrazole, m.p. 267-270°C.
d) The product from c) (3.0 g) was suspended in industrial methylated spirit (200 ml) and 5% palladium on charcoal (250 mg) was added followed by ammonium formate (2.05 g). The mixture was stirred and heated at 70°C for 3 hours and then cooled to ambient temperature and then filtered. The filtrate was concentrated under reduced pressure and triturated with dichloromethane to give 4-(1,4-dihydroindeno[1,2-c]pyrazol-3-yl)aniline, m.p. 253-254°C.
e) The product from d) (1.0 g) was dissolved in dichloromethane (30 ml) and triethyamine (0.62 ml) was added. The mixture was cooled to 0°C and benzene sulphonyl chloride (0.79 g) was added with stirring. The mixture was warmed to 20°C and stirred at this temperature for 2 hours. Further triethylamine (0.62 ml) and benzene sulphonyl chloride (0.79 g) were added and the mixture was stirred at ambient temperature for 4 hours and then allowed to stand at ambient temperature for 16 hours. Ether (80 ml) was added, followed by water (40 ml). The solid which precipitated was collected by filtration, washed with sodium bicarbonate solution and ether and then dried under vacuum at 60°C. The -material was recrystallized from acetone to give a solid which was purified by flash column chromatography on silica using dichloromethane to give a solid which was identified as 4'-(1-phenylsulphonyl(1,4-dihydroindeno[1,2-c]pyrazol-3-yl) *N*-phenylsulphonylaniline.
f) The product from e) (0.56 g) was suspended in methanol (40 ml) and 2M sodium hydroxide solution (5.3 ml) was.added. A clear solution was obtained and this was stirred at 20°C for 20 minutes. The mixture was poured into 2M hydrochloric acid (75 ml) and the solid obtained was collected by filtration to give a solid which was stirred with saturated sodium bicarbonate (25 ml) and ethyl acetate (25 ml) for 30 minutes and then filtered to give N-[4-(1,4-dihydroindeno[1,2-c]pyrazol-3-yl)phenyl]benzenesulphonamide, m.p. 286-288°C.

### Example 3

A mixture of the acid chloride from Example 1 d) (100 mg) dichloromethane (5 ml), 2-methoxyethanol (26 µl) and triethylamine (84 µl) was stirred at ambient temperature for 20 hours. The mixture was stirred with sodium bicarbonate solution (5 ml saturated) and filtered. The filtrate was evaporated to give a solid which was purified by flash column chromatography using dichloromethane/industrial methylated spirits (25:2) as the mobile phase to give 4-(1,4-dihydroindeno[1,2-c]pyrazol-3-yl)-N-(2-methoxyethyl)benzamide as a colourless solid.

### Example 4

A mixture of the acid chloride from Example 1 d) (100 mg), dichloromethane (5 ml), 4-nitroaniline (41 mg) and triethylamine (64 µl) was stirred at ambient temperature for 20 hours. The mixture was stirred with saturated sodium bicarbonate solution (5 ml) for 10 minutes and then filtered. The solid was washed with dichloromethane, then with alcohol and dried to give 4-(1,4-dihydroindeno[1,2-c]pyrazol-3-yl)-N-(4-nitrophenyl)benzamide as a colourless solid.

### Example 5

a) The aniline from Example 2 d) (6 g) was dissolved in dichloromethane (200 ml) and then triethylamine (7.4 ml) was added. The mixture was cooled to 0°C and then chloroacetyl chloride (4.2 ml) was added with stirring and the mixture was warmed to 20°C. The mixture was filtered and the solid obtained was washed with water and then ether and dried to give an intermediate which had been chloroacetylated on the 1-nitrogen of the pyrazole and on the NH₂ group of the starting aniline.
b) The product from a) (1.4 g), imidazole (0.95 g) and tetrahydrofuran (40 ml) were boiled under reflux at 90°C under nitrogen for 6 hours. The mixture was filtered and the filtrate was evaporated under reduced pressure to give a residue which was partitioned between ethyl acetate and 2M hydrochloric acid. The solid obtained was collected by filtration and dried to give 4-(1,4-dihydroindeno[1,2-c]pyrazol-3-yl)imidazol-1-ylacetanilide dihydrochloride, m.p. 264-266°C.

### Example 6

The product from Example 5 (0.27 g) was suspended in tetrahydrofuran (30 ml) under nitrogen with stirring and lithium aluminium hydride (87 mg) was added. The mixture was stirred at 20°C for 18 hours. The mixture was quenched with a saturated solution of sodium sulphate (40 ml) and then extracted with ethyl acetate (2 x 30 ml) to give a solid which was dissolved in ethanol (3 ml) to which concentrated hydrochloric acid (10 drops) was added. The ethanol was removed to give a yellow solid which was triturated with ether to give 4-(1,4-dihydroindeno[1,2-c]pyrazol-3-yl)-N-[2-(imidazol-1-yl)ethyl]aniline dihydrochloride, m.p. 205-209°C.

### Example 7

a) A mixture of 4-cyanobenzene sulphonyl chloride (5.15 g) in acetone (70 ml) was stirred at ambient temperature and then a solution of 2-morpholinoethylamine (6.7 ml) in acetone (15 ml) was added dropwise with stirring. The mixture was stirred at ambient temperature for 2 hours and then the acetone was removed by evaporation and the residue was eluted through a silica pad using ethyl acetate to give 4-cyano-*N*-2-morpholinoethylbenzenesulphonamide.
b) The product from a) (0.65 g) and dry toluene (30 ml) were stirred at 0-5°C and diisobutylaluminium hydride (4.4 ml of a 1.0M solution in cyclohexane) was added at 0-5°C. After the addition the solution was warmed to ambient temperature over 30 minutes and then warmed to boiling under reflux over 30 minutes and boiled for 3 hours. The mixture was cooled and added to 5M hydrochloric acid (10 ml) at 10-15°C. The mixture was then warmed to 90°C for 10 minutes and then cooled and the toluene layer was separated off. The aqueous layer was washed with ethyl acetate and then neutralized to pH 7-8 using 5M sodium hydroxide solution. This mixture was extracted with ethyl acetate, dried, filtered and evaporated to give a gum which was dried under vacuum at 40°C to give a solid which was identified as 4-formyl-*N*-(2-morpholinoethyl)benzene sulphonamide, m.p. 114-116°C.
c) The product from b) (200 mg), 2-bromoindanone (142 mg) and dry methanol (3 ml) were stirred at 0°C, and to this mixture was added a solution of sodium methoxide (44 mg) in methanol (0.5 ml). The mixture was stirred at 0°C for 2 hours to give a solid which was collected by filtration, washed with methanol and dried under vacuum to give the epoxide.
d) The product from c) (3.7 g), hydrazine hydrate (1 ml), glacial acetic acid (10 drops) and ethanol (100 ml) were boiled under reflux for 26 hours and the ethanol dried over molecular sieves in a Soxhlet extractor. The solvent was removed under reduced pressure and the residue was purified by flash column chromatography to give 4-(1,4-dihydroindeno[1,2-c]pyrazol-3-yl)-N-(2-morpholinoethyl)benzenesulphonamide, m.p. 218-220°C.

### Example 8

This was prepared in a similar manner to Example 7 except that 2-methoxyethylamine was used instead of 2-morphoninoethylamine and the product obtained was
4-(1,4-dihydroindeno[1,2-c]pyrazol-3-yl)-N-(2-methoxyethyl)benzenesulphonamide.

### Example 9

a) A mixture of indan-1-one (3.3 g), methyl 4-formylbenzoate (5.0 g), piperidine (0.6 ml) and glacial acetic acid (0.5 ml) was heated on a steam bath for 3 hours. The solid mass obtained was boiled up in industrial methylated spirits (200 ml) and then hot filtered. The solid residue obtained was washed with industrial methylated spirits and dried to give methyl 4-(1-oxoindan-2-ylidenemethyl)benzoate, m.p. 194-198°C.
b) The product from a) (1.5 g) was suspended in methanol (10 ml) and dichloromethane (15 ml) and stirred at 0-5°C whilst 2M sodium hydroxide solution (2.7 ml) was added followed by 30% hydrogen peroxide (100 vol. 1.1 ml). The mixture was stirred at 0-5°C for 5 minutes then at ambient temperature for 24 hours. Dichloromethane (100 ml) was added to the mixture which was then washed with brine (2 x 50 ml), dried, filtered and evaporated to give methyl 4-(1-oxospiro[indan-2,2'-oxiran]-3'-yl)benzoate, m.p. 160-163°C. The aqueous phase was acidified with 5M hydrochloric acid and extracted with dichloromethane to give 4-(1-oxospiro[indan-2,2'-oxiran]-3'-yl)benzoic acid, m.p. 220°C with decomposition.
c) A mixture of methyl 4-(1-oxospiro[indan-2,2'-oxiran]-3'-yl)benzoate (750 mg), methanol (30 ml) and hydrazine hydrate (0.16 ml) was stirred at ambient temperature whilst glacial acetic acid (6 drops) was added. The mixture was boiled under reflux for 24 hours and then allowed to stand at ambient temperature for 24 hours, then cooled to 0°C and filtered to give methyl 4-(1,4-dihydroindeno[1,2-c]pyrazol-3-yl)benzoate, m.p. 224-226°C.
d) The ester from c) (2.20 g) and *N*,*N*-diethylethylenediamine (7 ml) were boiled under reflux for 4.5 hours. The mixture was cooled to ambient temperature and then petroleum ether, b.p. 40-60°C (50 ml) was added. The mixture was filtered to give the amide.
e) The amide (2.75 g) was suspended in tetrahydrofuran (80 ml) and stirred at ambient temperature under nitrogen as lithium aluminium hydride (1.14 g) was added. The mixture was stirred for 3.5 hours and then further lithium aluminium hydride (1.14 g) and tetrahydrofuran (40 ml) were added. A third portion of lithium aluminium hydride was added after 22.5 hours and this mixture was then stirred for 24 hours. The mixture was boiled under reflux for 2.5 hours then stood overnight at ambient temperature. The mixture was stirred under nitrogen with cooling in an ice-bath while ethyl acetate (100 ml) was added, followed by water (100 ml) the organic layer was separated off, washed, dried and evaporated to give an oil which was purified by flash column chromatography on silica using ethyl acetate/ethanol/triethylamine (7:2:1). Appropriate fractions were combined and evaporated to give a gum (0.85 g) which was dissolved in ethanol (5 ml) with warming and to the solution was added concentrated hydrochloric acid (0.6 ml). The mixture was then evaporated under reduced pressure and the residual gummy solid was boiled with ethanol (10 ml) then cooled in ice and filtered to give N-[2-(N,N-diethylamino)ethyl]-4-(1,4-dihydroindeno[1,2-c]pyrazol-3-yl)benzylamine trihydrochloride, m.p. 225°C.

### Example 10

This example was prepared in a similar manner to Example 7 except that *N,N*-diethylaminoethylamine was used instead of 2-morpholinoethyl amine. The product obtained was N-[2-(N,N-diethylamino)ethyl]-4-(1,4-dihydroindeno[1,2-c]pyrazol-3-yl)benzenesulphonamide dihydrochloride, m.p. 192-196°C.

### Example 11

This example was prepared in a similar manner to Example 9 using 2-morpholinoethylamine instead of *N*,*N*-diethylethylenediamine. The product obtained was 4-(1,4-dihydroindeno[1,2-c]pyrazol-3-yl)-N-(2-morpholinoethyl)benzylamine dihydrochloride, m.p. 266-269°C (with decomposition).

### Example 12

This was prepared in a similar manner to Example 1 except that 4-ethoxyaniline was used instead of 2-methoxyethylamine. The product obtained was N-(4 -ethoxyphenyl)-4-(1,4-dihydroindeno[1,2-c]pyrazol-3-yl)benzylamine, m.p. 180°C (with decomposition).

### Example 13

This was prepared in a similar manner to Example 9 d) except that (S-(+)-2-(aminomethyl)pyrrolidine was used instead of *N*,*N*-diethylethylenediamine. The product obtained was (S)-4-(1,4-dihydroindeno[1,2-c]pyrazol-3-yl)-N-(pyrrolidin-2-ylmethyl)benzamide dihydrochloride, m.p. 222-226°C.

### Example 14

a) Methyl thiosalicylate (9.89 ml) was added to a solution of sodium methoxide (11.6 g) in ethanol (100 ml) with stirring. After 15 minutes a solution of 4'-bromophenacyi bromide (20.0 g) in ethanol (100 ml) was added and the mixture was stirred and boiled under reflux for 18 hours. The mixture was cooled and acidified with 10% hydrochloric acid (150 ml). The solid was collected and used directly in the next experiment.
b) The product from a) (18.0 g) was stirred and boiled under reflux in ethanol (150 ml) in a flask fitted with a Soxhlet extractor thimble containing 4Å molecular sieves. 1 Drop of glacial acetic acid was added followed by hydrazine hydrate (3.9 ml) and the mixture was boiled and stirred under reflux for 64 hours. The mixture was cooled and the precipitate was collected and used directly in the next experiment.
c) The product from b) (4.0 g) was dissolved in tetrahydrofuran (200 ml) and added dropwise with stirring at 0°C under nitrogen to a stirred suspension of potassium hydride (1.53 g) in tetrahydrofuran (100 ml). After the addition the mixture was stirred for 15 minutes and then cooled to -78°C. *Tert*-butyl lithium (17.0 ml of a 1.5M solution in pentane) was added dropwise and after stirring for 45 minutes at this temperature dimethylformamide (4.7 ml) was added. The temperature was maintained at -78°C for 1 hour and then the reaction mixture was allowed to warm slowly to ambient temperature. The mixture was quenched by the careful addition of 1M hydrochloric acid (200 ml). The organic layer was separated, washed with water, saturated bicarbonate, brine and then dried, filtered and evaporated to give a waxy red solid which was purified by flash column chromatography on silica using 20% ethyl acetate in petroleum ether, b.p. 260-280°C as the mobile phase to give 3-(4-formylphenyl)-1H-[1]benzothieno[3,2-c]pyrazole, m.p. 261-263°C.
d) A solution of the product from c) (100 mg), 3-(1-imidazolyl)propylamine (58 mg) in 1,2-dichloroethane containing glacial acetic acid (0.03 ml) was stirred at ambient temperature for 1 hour. Sodium triacetoxyborohydride (84 mg) was added and the mixture was stirred at ambient temperature for 16 hours. Additional sodium triacetoxyborohydride (90 mg) was added and the mixture was stirred at ambient temperature for 24 hours. The mixture was poured into a stirred solution of saturated aqueous sodium bicarbonate (approx. 20 ml). The organic layer was separated and the aqueous layer was extracted with dichloromethane. The combined organic layer and extracts were washed with water, dried and evaporated under reduced pressure to give a solid which was dissolved in ethanol (15 ml) and 2 drops of concentrated hydrochloric acid were added.The solution was concentrated under reduced pressure to give a solid which was triturated with diethylether, filtered and dried to give 4-(1H-[1]benzothieno[3,2-c]pyrazol-3-yl)-N-[3-(imidazol-1-yl)propyl]benzylamine trihydrochloride, m.p. 206-208°C (with decomposition).

### Example 15

This example was prepared in a similar manner to Example 14 by reacting 3-(4-formylphenyl)-1H-[1]benzothieno[3,2-c]pyrazole with 2-morpholinoethylamine to give 4-(1H-[1]benzothieno[3,2-c]pyrazol-3-yl)-N-(2-morpholinoethyl)benzylamine trihydrochloride, m.p. 270-272°C.

### Example A

The use of compounds of the present invention in the manufacture of pharmaceutical compositions is illustrated by the following description. In this description the term "active compound" denotes any compound of the invention but particularly any compound which is the final product of one of the preceding Examples.

### a) Capsules

In the preparation of capsules, 10 parts by weight of active compound and 240 parts by weight of lactose are de-aggregated and blended. The mixture is filled into hard gelatin capsules, each capsule containing a unit dose or part of a unit dose of active compound.

### b) Tablets

Tablets are prepared from the following ingredients.

| | Parts by weight |
|---|---|
| Active compound | 10 |
| Lactose | 190 |
| Maize starch | 22 |
| Polyvinylpyrrolidone | 10 |
| Magnesium stearate | 3 |

The active compound, the lactose and some of the starch are de-aggregated, blended and the resulting mixture is granulated with a solution of the polyvinylpyrrolidone in ethanol. The dry granulate is blended with the magnesium stearate and the rest of the starch. The mixture is then compressed in a tabletting machine to give tablets each containing a unit dose or a part of a unit dose of active compound.

### c) Enteric coated tablets

Tablets are prepared by the method described in (b) above. The tablets are enteric coated in a conventional manner using a solution of 20% cellulose acetate phthalate and 3% diethyl phthalate in ethanol:dichloromethane (1:1).

### d) Suppositories

In the preparation of suppositories, 100 parts by weight of active compound is incorporated in 1300 parts by weight of triglyceride suppository base and the mixture formed into suppositories each containing a therapeutically effective amount of active ingredient.

### EQUIVALENTS

While this invention has been particularly shown and described with references to preferred embodiments thereof, it will be understood by those skilled in the art that various changes in form and details may be made therein without departing from the scope of the invention as defined by the appended claims. Those skilled in the art will recognise or be able to ascertain using no more than routine experimentation, many equivalents to the specific embodiments of the invention described specifically herein. Such equivalents are intended to be encompassed in the scope of the claims.

## Claims

1. Compounds formula I 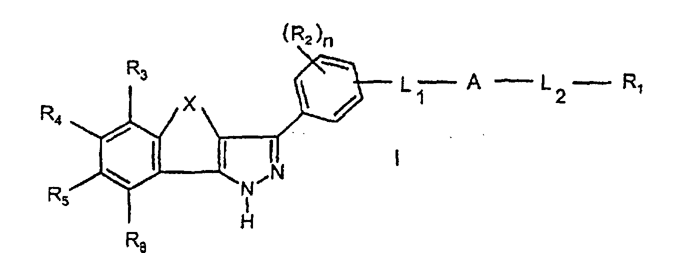 and pharmaceutically acceptable salts thereof in which
L₁ represents a group of formula (E)ₛ(CH₂)_{q} in which E represents NR₂₄, O or S, s is 0 or 1 and q is an integer from 0 to 6, provided that when s is 1 q is at least 1, in which the alkylene chain is optionally substituted by one or more of the following: a C₁₋₆ alkyl group optionally substituted by one or more hydroxy, halo or optionally substituted amino; a C₁₋₆ alkoxy group optionally substituted by one or more hydroxy, halo or optionally substituted amino; hydroxy; halo; or optionally substituted amino;
A represents CONH, NHCO, SO₂NH, NHSO₂, or NR₂₅;
L₂ represents a group of formula (CH₂)ᵣ in which r is an integer from 0 to 6 in which the alkylene chain is optionally substituted by one or more of the following: a C₁₋₆ alkyl group optionally substituted by one or more hydroxy, halo or optionally substituted amino; a C₁₋₆ alkoxy group optionally substituted by one or more hydroxy, halo or optionally substituted amino; hydroxy; halo; or optionally substituted amino;
R₂ represents a C₁₋₆ alkyl group optionally substituted by one or more of the following: halo, hydroxy, C₁₋₆ alkoxy or optionally substituted amino; a C₁₋₆ alkoxy group optionally substituted by one or more of the following: halo, hydroxy, C₁₋₆ alkoxy or optionally substituted amino ; or R₂ is halo, hydroxy, cyano, nitro, carbamoyl, a C₁₋₆ alkanoylgroup, a (C₁₋₆ alkoxy)carbonyl group or optionally substituted amino;
n represents 0,1,2 or 3
X represents a) substituted methylene b) carbonyl, c) oxygen , d) a group of formula -C=NOR₇ in which R₇ represents H or a C₁₋₄ alkyl group, e) a group of formula NR₈ in which R₈ represents H, an optionally substituted C₁₋₄ alkyl group or optionally substituted phenyl, f) a group of formula (CH₂)ₙ in which n is 1, 2 or 3, or g) a group of formula S(O)ₚ in which p is 0, 1 or 2;
R₃, R₄ , R₅ and R₆ independently represent a) H, b) halo, c) a C₁₋₆ alkyl group optionally substituted by one or more of the following: hydroxy, a C₁₋₆ alkoxy group, halo or an optionally substituted amino group d) a C₁₋₆ alkoxy group optionally substituted by one or more of the following: hydroxy; a C₁₋₆ alkoxy group; halo; or an optionally substituted amino group provided that these groups are not attached to the carbon which is attached to the oxygen of the alkoxy group; e) optionally substituted phenoxy, f) hydroxy, g) a group formula CORₐ in which Rₐ represents hydroxy, a C₁₋₆ alkoxy group or Rₐ represents an optionally substituted amino group, h) an optionally substituted amino group i) a C₁₋₆ alkanoyl group j) nitro, k) optionally substituted phenyl C₁₋₆ alkyl, l) optionally substituted phenyl C₁₋₆ alkoxy m) cyano; or o) a C₂₋₄ alkenyl group or a C₂₋₄ alkynyl group each of which is optionally substituted by phenyl which is optionally substituted by one or more of the following : a C₁₋₆ alkyl group, a C₁₋₆ alkoxy group or halo;
and
1) when A is SO₂NH, or NHSO₂
R₁ represents a) optionally substituted phenyl b) optionally substituted heteroaryl, c) a five, six or seven membered saturated heterocyclic ring containing a nitrogen atom which optionally contains an additional hetero atom selected from O, S or N and is optionally substituted by a C₁₋₆ alkyl group wherein said saturated ring may be attached through carbon or a hetero atom d) an optionally substituted amino group or e) a C₁₋₆ alkoxy group;
2) when A represents CONH or NHCO
R₁ represents a) phenyl substituted by nitro or one or more C₁₋₆ alkoxy groups optionally substituted by one or more of the following: halo, hydroxy, C₁₋₆ alkoxy or optionally substituted amino b) optionally substituted heteroaryl or c) a five, six or seven membered saturated heterocyclic ring containing a nitrogen atom which optionally contains an additional hetero atom selected from O, S or N and is optionally substituted by a C₁₋₆ alkyl group wherein said saturated ring is attached through a carbon atom or d) a C₁₋₆ alkoxy group;
3) when A represents a group NR₂₅ and q is at least 1 then
R₁ represents a) optionally substituted phenyl b) optionally substituted heteroaryl or c) an optionally substituted amino group; and
4) when A represents a group NR₂₅ and q is 0 and s is 0 then R₁ represents optionally substituted heteroaryl;
R₂₄ and R₂₅ independently represent H; a C₁₋₆ alkyl group optionally substituted by one or more of the following: hydroxy, a C₁₋₆ alkoxy group, halo or an optionally substituted amino group; a C₁₋₆ alkanoyl group or a C₁₋₆ alkylsulphonyl group; provided that no two hetero atoms are attached to the same sp3 hybridized carbon atom,
wherein substituted methylene means methylene substituted by one or more of hydroxy or a C₁₋₄ alkyl group wherein the alkyl group is optionally further substituted by a group of formula NRᵣRₛ wherein Rr and Rs independently represent H or a C₁₋₆ alkyl group;
wherein optionally substituted amino group means a group of formula NRₖRₗ in which Rₖ and Rₗ independently represent hydrogen, a C₁₋₁₂ alkyl group, a C₁₋₁₂ alkoxy group, a C₃₋₁₂ cycloalkyl group, a phenyl group, a phenylC₁₋₆ alkyl group, a heteroaryl group or a heteroarylC₁₋₆ alkyl group wherein each of said groups is optionally substituted by one or more of the following: a C₁₋₆ alkyl group, a C₁₋₆ alkoxy group, hydroxy, halo; or Rₖ and Rₗ together with the nitrogen atom to which they are attached represent a five, six or seven membered saturated heterocyclic ring which optionally contains an additional hetero atom selected from O, S or N and is optionally substituted by a C₁₋₆ alkyl group;
wherein heteroaryl means an optionally substituted mono or bicyclic aromatic heterocycle in which the heterocycle contains 1.2,3 or 4 hetero atoms selected from nitrogen, sulphur or oxygen;
wherein optionally substituted phenyl and heteroaryl means phenyl and heteroaryl substituted by one or more of a) halo, b) a C₁₋₆ alkyl group optionally substituted by one or more of hydroxy, halo, an optionally substituted amino group or a five, six or seven membered saturated heterocyclic ring containing a nitrogen atom which optionally contains an additional hetero atom selected from O, S or N and is optionally substituted by a C₁₋₆ alkyl group wherein said saturated ring is attached through a carbon atom, c) a C₁₋₆ alkoxy group optionally substituted by one or more of hydroxy; a C₁₋₆ alkoxy group; halo; or optionally substituted amino group provided that these groups are not attached to the carbon which is attached to the oxygen of the alkoxy group, or a five, six or seven membered saturated heterocyclic ring containing a nitrogen atom which optionally contains an additional hetero atom selected from O, S or N and is optionally substituted by a C₁₋₆ alkyl group wherein said saturated ring is attached through a carbon atom, d) optionally substituted phenoxy, e) hydroxy, f) a group Formula CORₐ in which Rₐ represents hydroxy, a C₁₋₆ alkoxy group or Rₐ represents a group of formula NR_{b} R_{c} in which R_{b} and R_{c} independently represent hydrogen, a C₁₋₁₂ alkyl group , a C₃₋₁₂ cycloalkyl group or phenyl wherein the alkyl group, the cycloalkyl group and phenyl are optionally substituted by one or more of hydroxy, halo, a C₃₋₁₂ cycloalkyl group or an amino group of formula NRₕRⱼ wherein Rₕ and Rⱼ independently represent hydrogen or a C₁₋₆ alkyl group or wherein Rₕ and Rⱼ together with the nitrogen atom to which they are attached represent a five, six or seven membered saturated heterocyclic ring which optionally contains an additional hetero atom selected from O, S or N and is optionally substituted by a C₁₋₆ alkyl group, g) a group of formula NR_{d} R_{c} in which R_{d} and R_{c} are independently selected from hydrogen, a C₁₋₁₂ alkyl group , a C₃₋₁₂ cycloalkyl group or phenyl or a group of formula COR_{f} wherein R_{f} represents hydrogen, a C₁₋₁₂ alkyl group, a C₃₋₁₂ cycloalkyl group, a phenyl C₁₋₆ alkyl group orphenyl wherein in each case the alkyl group, the cycloalkyl group and phenyl are optionally substituted by one or more of halo, hydroxy, nitro or an amino group of formula NRₕRⱼ wherein Rₕ and Rⱼ are as defined above, h) a group of fonnula O(CH₂)ₘ R_{g} in which m is 2, 3, 4 or 5 and R_{g} represents hydroxy or a group of formula NR_{d}R_{c} in which R_{d} and R_{c} are as defined above; or R_{g} represents a group of formula CORₐ wherein Rₐ is as defined above and m is 1,2,3,4 or 5, i) nitro, j) optionally substituted phenyl C₁₋₆ alkyl, k) optionally substituted phenyl C₁₋₆ alkoxy, l) cyano, m) a C₃₋₆alkenyloxy group, n) a pyridyloxy or pyridylthio group in which the pyridine ring is optionally substituted by one or more of the following : trifluoromethyl or nitro, o) hydroxyamidino, p) aminomethyl, q) formamidomethyl, r) a C₁₋₆ alkythio group, s) phenyl or t) a C₂-₄ alkenyl group or a C₂₋₄ alkynyl group each of which is optionally substituted by phenyl which is optionally substituted by one or more of the following : a C₁₋₆ alkyl group, a C₁₋₆ alkoxy group or halo.

2. Compounds according to Claim 1 in which X is CH₂ or S.

3. Compounds according to Claim 1 in which X is CH₂ and A is NR₂₅.

4. Compounds according to Claim 1 in which X is S and A is NR₂₅.

5. Compounds according to Claim 1 in which X is CH₂ and A is HNSO₂.

6. Compounds according to Claim 1 in which X is CH₂ and A is SO₂NH.

7. Compounds according to Claim 1 in which X is CH₂ and A is CONH.

8. Compounds according to Claim 1 in which X is CH₂ and A is HNCO.

9. Compounds according to claim 1 in which X is CH₂, A is NR₂₅, L₁ is (CH₂)_{q} in which q is an integer from 1 to 6 and the alkylene chain is optionally substituted by one or more of the following: a C₁₋₆ alkyl group optionally substituted by one or more hydroxy, halo or optionally substituted amino; a C₁₋₆ alkoxy group optionally substituted by one or more hydroxy, halo or optionally substituted amino; hydroxy; halo; or optionally substituted amino; L₂ is a bond and R₁ is optionally substituted pyridyl.

10. A compound selected from:
4-(1,4-dihydroindeno[1,2-c]pyrazole)-N-(4-pyridyl)benzylamine
N-[4-(1,4-dihydroindeno[1,2-c]pyrazol-3-yl)phenyl]benzenesulphonamide
4-(1,4-dihydroindeno[1,2-c]pyrazol-3-yl)-N-(2-methoxyethyl)benzamide
4-(1,4-dihydroindeno[1,2-c]pyrazol-3-yl)-N-(4-nitrophenyl)benzamide
4-(1,4-dihydroindeno[1,2-c]pyrazol-3-yl)imidazol-1-ylacetanilide
4-(1,4-dihydroindeno[1,2-c]pyrazol-3-yl)-N-[2-(imidazol-1-yl)ethyl]aniline
4-(1,4-dihydroindeno[1,2-c)pyrazol-3-yl)-N-(2-morpholinoethyl)benzenesulphonamide
4-(1,4-dihydroindeno[1,2-c]pyrazol-3-yl)-N-(2-methoxyethyl)benzenesulphonamide
N-[2-(N,N-diethylamino)ethyl]-4-(1,4-dihydroindeno[1,2-c]pyrazol-3-yl)benzylamine
N-[2-(N,N-diethylamino)ethyl]-4-(1,4-dihydroindeno[1,2-c]pyrazol-3-yl)benzene-sulphonamide
4-(1,4-dihydroindeno[1,2-c]pyrazol-3-yl)-N-(2-morpholinoethyl)benzylamine
N-(4-ethoxyphenyl)-4-(1,4-dihydroindeno[1,2-c]pyrazol-3-yl)benzylamine
(S)-4-(1,4-dihydroindeno[1,2-c]pyrazol-3-yl)-N-(pyrrolidin-2-ylmethyl)benzamide
4-(1H-[1]benzothieno[3,2-c]pyrazol-3-yl)-N-[3-(imidazol-1-yl)propyl]benzylamine
4-(1H-[1]benzothieno[3,2-c]pyrazol-3-yl)-N-(2-morpholinoethyl)benzylamine
and pharmaceutically acceptable salts thereof including individual enantiomers and mixture of enantiomers.

11. A pharmaceutical composition comprising a compound of formula 1 as defined in claim 1 in conjunction with a pharmaceutically acceptable diluent or carrier.

12. A compound of formula I as defined in claim 1 for use as a medicament.

13. A compound of formula I as defined in claim 1 for use as a medicament for inhibiting protein kinase activity.

14. The use of a compound of formula I as defined in claim I in the manufacture of a medicament for inhibiting protein kinase activity.

15. The use of Claim 14 wherein said protein kinase is a tyrosine kinase.

16. The use of Claim 15 wherein said tyrosine kinase is either a receptor tyrosine kinase or a non-receptor tyrosine kinase.

17. The use of Claim 16 wherein said tyrosine kinase is selected from the group consisting of KDR, flt-1, TIE-2, Lck, Src, fyn and yes.

18. The use of Claim 14 wherein the activity of said tyrosine kinase affects angiogenesis.

19. The use of Claim 18 wherein the inhibition of said tyrosine kinase is anti-angiogenic.

20. The use of Claim 19 wherein said inhibition of said tyrosine kinase inhibits the progression of a disease state selected from the group consisting of cancer, arthritis, atherosclerosis, psoriasis, hemangioma, myocardial angiogenesis, coronary and cerebral collateral vascularization, ischemic limb angiogenesis, corneal disease, rubeosis, neovascular glaucoma, macular degeneration, wound healing, peptic ulcer *Helicobacter* related diseases, fractures, diabetic retinopathy, and cat scratch fever.

21. The use of Claim 15 wherein the activity of said tyrosine kinase affects vascular hyperpermeability or the production of edema.

22. The use of Claim 21, wherein the inhibition of said tyrosine kinase is antiedematous.

23. The use of Claim 22, wherein the inhibition of said tyrosine kinase inhibits the progression of a disease state selected from the group consisting of burns, chronic lung disease, stroke, polyps, psoriasis, allergic inflammation, macular degeneration, diabetic retinopathy, ovarian hyperstimulation syndrome and brain tumor-associated cerebral edema.

24. The use of Claim 15 wherein the inhibition of said tyrosine kinase has an anti-tumor effect.

25. The use of Claim 15 wherein the inhibition of said tyrosine activity is associated with anti-fertility or abortifacient effects.

26. The use of Claim 14 wherein said protein kinase is a serine kinase.

27. The use of Claim 14 wherein said protein kinasc is a threonine kinase.

28. The use of Claim 15 wherein said tyrosine kinase is KDR.

## Patentansprüche

1. Verbindungen der Formel I 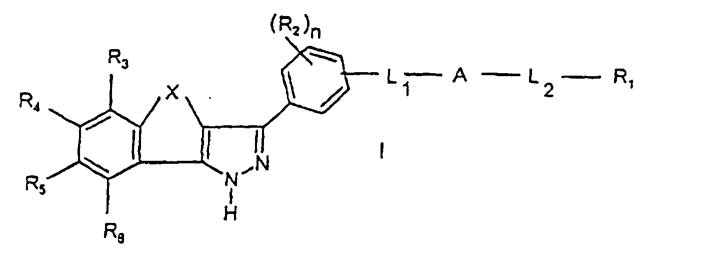 und pharmazeutisch verträgliche Salze davon, in denen
L₁ eine Gruppe der Formel (E)ₛ(CH₂)_{q} darstellt, in der E NR₂₄, O oder S darstellt, s ist 0 oder 1 und q ist eine ganze Zahl von 0 bis 6, vorausgesetzt daß, wenn s 1 ist, q mindestens 1 ist, in der die Alkylenkette wahlweise sustituiert ist durch ein oder mehrere der folgenden: eine C₁-C₆ Alkylgruppe, wahlweise substituiert durch ein oder mehrere von Hydroxy, Halo oder wahlweise substituiertem Amino; eine C₁₋₆ Alkoxygruppe wahlweise substituiert durch ein oder mehrere von Hydroxy, Halo oder wahlweise substituiertem Amino; Hydroxy; Halo; oder wahlweise substituiertes Amino;
A stellt CONH, NHCO, SO₂NH, NHSO₂ oder NR₂₅ dar;
L₂ stellt eine Gruppe der Formel (CH₂)ᵣ dar, in der r eine ganze Zahl von 0 bis 6 ist, in der die Alkylenkette wahlweise substituiert ist durch ein oder mehrere der folgenden: eine C₁-₆ Alkylgruppe wahlweise substituiert durch ein oder mehrere von Hydroxy, Halo oder wahlweise substituiertem Amino; einer C₁-₆ Alkoxygruppe wahlweise substituiert durch ein oder mehrere von Hydroxy, Halo oder wahlweise substituiertem Amino; Hydroxy; Halo; oder wahlweise substituiertes Amino;
R₂ stellt eine C₁-₆ Alkylgruppe dar, wahlweise substituiert durch ein oder mehrere der folgenden: Halo, Hydroxy, C₁-₆ Alkoxy oder wahlweise substituiertes Amino, eine C₁₋₆ Alkoxygruppe wahlweise substituiert durch ein oder mehrere der folgenden: Halo, Hydroxy, C₁₋₆ Alkoxy oder wahlweise substituiertes Amino; oder R₂ ist Halo, Hydroxy, Cyano, Nitro, Carbamoyl, eine C₁₋₆ Alkanoylgruppe, eine (C₁₋₆ Alkoxy)carbonylgruppe oder wahlweise substituiertes Amino;
n stellt 0, 1, 2 oder 3 dar,
X stellt folgendes dar: a) sustituiertes Methylen, b) Carbonyl, c) Sauerstoff, d) eine Gruppe der Formel -C=NOR₇, in der R₇ H oder eine C₁₋₄ Alkylgruppe darstellt, e) eine Gruppe der Formel NR₈ in der R₈ H darstellt, eine wahlweise substituierte C₁₋₄ Alkylgruppe oder wahlweise substituiertes Phenyl, f) eine Gruppe der Formel (CH₂)ₙ in der n 1, 2 oder 3 ist, oder g) eine Gruppe der Formel S(O)ₚ, in der p 0, 1 oder 2 ist;
R₃, R₄, R₅ und R₆ stellen unabhängig folgendes dar: a) H, b) Halo, c) eine C₁₋₆ Alkylgruppe, wahlweise substituiert durch ein oder mehrere der folgenden: Hydroxy, eine C₁₋₆ Alkoxygruppe, Halo oder eine wahlweise substituierte Aminogruppe, d) eine C₁₋₆ Alkoxygruppe wahlweise substituiert durch ein oder mehrere der folgenden: Hydroxy; eine C₁₋₆ Alkoxygruppe; Halo; oder eine wahlweise substituierte Aminogruppe, vorausgesetzt, daß diese Gruppen nicht an den Kohlenstoff gebunden sind, der an den Sauerstoff der Alkoxygruppe. gebunden ist; e) wahlweise substituiertes Phenoxy, f) Hydroxy, g) eine Gruppe der Formel CORₐ, in der Rₐ Hydroxy, eine C₁₋₆ Alkoxygruppe darstellt, oder Rₐ stellt eine wahlweise substituierte Aminogruppe dar, h) eine wahlweise substituierte Aminogruppe, i) eine C₁₋₆ Alkanoylgruppe, j) Nitro, k) wahlweise substituiertes Phenyl C₁₋₆ Alkyl, l) wahlweise substituiertes Phenyl C₁₋₆ Alkoxy, m) Cyano; oder o) eine C₂₋₄ Alkenylgruppe oder eine C₂₋₄ Alkinylgruppe, die jeweils wahlweise substituiert ist durch Phenyl, das wahlweise subtituiert ist durch ein oder mehrere der folgenden: eine C₁₋₆ Alkylgruppe, eine C₁₋₆ Alkoxygruppe oder Halo; und
1) wenn A SO₂NH oder NHSO₂ ist
dann stellt R₁ folgendes dar: a) wahlweise substituiertes Phenyl, b) wahlweise substituiertes Heteroaryl, c) einen fünf-, sechsoder sieben-gliedrigen gesättigten heterozyklischen Ring enthaltend ein Stickstoffatom, das wahlweise ein zusätzliches Heteroatom enthält, gewählt aus O, S oder N, und wahlweise substituiert ist durch eine C₁-₆ Alkylgruppe, worin der gesättigte Ring gebunden sein kann durch Kohlenstoff oder ein Heteroatom, d) eine wahlweise substituierte Aminogruppe oder e) eine C₁-₆ Alkoxygruppe;
2) wenn A CONH oder NHCO darstellt,
R₁ stellt folgendes dar: a) Phenyl substituiert durch Nitro oder ein oder mehrere C₁-₆ Alkoxygruppen wahlweise substituiert durch ein oder mehrere der folgenden: Halo, Hydroxy, C₁-₆ Alkoxy oder wahlweise substituiertes Amino, b) wahlweise sustituiertes Heteroaryl oder c) einen fünf-, sechs- oder sieben-gliedrigen gesättigten heterozyklischen Ring, enthaltend ein Stickstoffatom, der wahlweise ein zusätzliches Heteroatom enthält, gewählt aus O, S oder N und wahlweise substituiert ist durch eine C₁-₆ Alkylgruppe, worin der gesättigte Ring durch ein Kohlenstoffatom gebunden ist oder d) eine C₁-₆ Alkoxygruppe;
3) wenn A eine Gruppe NR₂₅ darstellt und q mindestens 1 ist, dann stellt R₁ a) wahlweise substituiertes Phenyl, b) wahlweise substituiertes Heteroaryl oder c) eine wahlweise substituierte Aminogruppe dar; und
4) wenn A eine Gruppe NR₂₅ darstellt und q 0 ist und s 0 ist, dann stellt R₁ ein wahlweise substituiertes Heteroaryl dar;
R₂₄ und R₂₅ stellt unabhängig H dar; eine C₁-C₆ Alkylgruppe wahlweise substituiert durch ein oder mehrere der folgenden: Hydroxy, eine C₁-₆ Alkoxygruppe, Halo oder eine wahlweise substituierte Aminogruppe; eine C₁-₆ Alkanoylgruppe oder eine C₁-₆ Alkylsulphonylgruppe; vorausgesetzt, daß keine zwei Heteroatome an das gleiche sp3 hybridisierte Kohlenstoffatom gebunden sind,
worin substituiertes Methylen Methylen bedeutet, substituiert durch ein oder mehrere von Hyrdoxy oder eine C₁-₄ Alkylgruppe,
worin die Alkylgruppe weiter wahlweise substituiert ist durch eine Gruppe der Formel NRᵣRₛ, worin Rᵣ und Rₛ unabhängig H oder eine C₁-₆ Alkylgruppe darstellen;
worin eine wahlweise substituierte Aminogruppe eine Gruppe der Formel NRₖRₗ bedeutet, in der Rₖ und R₁ unabhängig Wasserstoff, eine C₁-₁₂ Alkylgrupe, eine C₁-₁₂ Alkoxygruppe, eine C₃-₁₂ Cycloalkylgruppe, eine Phenylgruppe, eine Phenyl C₁-₆ Alkylgruppe, eine Heteroarylgruppe oder eine Heteroaryl C₁-₆ Alkylgruppe darstellt, worin jede der Gruppen wahlweise substituiert ist durch ein oder mehrere der folgenden: eine C₁-₆ Alkylgruppe, eine C₁-₆ Alkoxygruppe, Hydroxy, Halo; oder Rₖ und R₁ zusammen mit dem Stickstoffatom, an das sie gebunden sind, stellen einen fünf-, sechs- oder sieben-gliedrigen gesättigten heterozyklischen Ring dar, der wahlweise ein zusätzliches Heteroatom enthält, gewählt aus O, ⁻S oder N und ist wahlweise substituiert durch eine C₁₋₆ Alkylgruppe;
worin Heteroaryl einen wahlweise substituierten mono- oder bizyklischen aromatischen Heterozyklus bedeutet, in dem der Heterozyklus 1, 2, 3 oder 4 Heteroatome enthält, gewählt aus Stickstoff, Schwefel oder Sauerstoff;
worin wahlweise substituiertes Phenyl und Heteroaryl Phenyl und Heteroaryl bedeuten, substituiert durch ein oder mehrere von a) Halo, b) einer C₁₋₆ Alkylgruppe wahlweise substituiert durch ein oder mehrere von Hydroxy, Halo, einer wahlweise substituierten Aminogruppe oder einem fünf-, sechs- oder sieben-gliedrigen gesättigten heterozyklischen Ring, enthaltend ein Stickstoffatom, der wahlweise ein zusätzliches Heteroatom enthält, gewählt aus O, S oder N, und der wahlweise substituiert ist durch eine C₁₋₆ Alkylgruppe, worin der gesättigte Ring durch ein Kohlenstoffatom gebunden ist, c) eine C₁-₆ Alkoxygruppe wahlweise substituiert durch ein oder mehrere von Hydroxy; eine C₁-₆ Alkoxygruppe; Halo; oder eine wahlweise substituierte Aminogruppe, vorausgesetzt, daß diese Gruppen nicht an den Kohlenstoff gebunden sind, der an den Sauerstoff der Alkoxygruppe gebunden ist, oder ein fünf-, sechs- oder siebengliedriger gesättiger heterozyklischer Ring ist, enthaltend ein Stickstoffatom, der wahlweise ein zusätzliches Heteroatom enthält, gewählt aus O, S oder N, und wahlweise substituiert ist durch eine C₁-₆ Alkylgruppe, worin der gesättigte Ring durch ein Kohlenstoffatom gebunden ist, d) wahlweise substituiertes Phenoxy, e) Hydroxy, f) eine Gruppe der Formel CORₐ, in der Rₐ Hydroxy, eine C₁-₆ Alkoxygruppe darstellt oder Rₐ stellt eine Gruppe der Formel NR_{b}R_{c} dar, in der R_{b} und R_{c} unabhängig Wasserstoff, eine C₁-₁₂ Alkylgruppe, eine C₃-₁₂ Cycloalkylgruppe oder Phenyl darstellen, worin die Alkylgruppe, die Cycloalkylgruppe und Phenyl wahlweise substituiert sind durch ein oder mehrere von Hydroxy, Halo, einer C₃₋₁₂ Cycloalkylgruppe oder eine Aminogruppe der Formel NRₕRⱼ, worin Rₕ und Rⱼ unabhängig Wasserstoff oder eine C₁₋₆ Alkylgruppe darstellen, oder worin Rₕ und Rⱼ zusammen mit dem Stickstoffatom, an welches sie gebunden sind einen fünf-, sechs- oder sieben-gliedrigen gesättigten heterozyklischen Ring darstellen, der wahlweise ein zusätzliches Heteroatom enthält, gewählt aus O, S oder N, und wahlweise substituiert ist durch eine C₁-₆ Alkylgruppe, g) eine Gruppe der Formel NR_{d}Rₑ, in der R_{d} und Rₑ unabhängig gewählt sind aus Wasserstoff, einer C₁-₁₂ Alkylgruppe, einer C₃-₁₂ Cycloalkylgruppe oder Phenyl oder eine Gruppe der Formel COR_{f}, worin R_{f} Wasserstoff, eine C₁-₁₂ Alkylgruppe, eine C₃-C₁₂ Cycloalkylgruppe, eine Phenyl C₁-₆ Alkylgruppe oder Phenyl darstellen, worin in jedem Fall die Alkylgruppe, die Cycloalkylgruppe und das Phenyl wahlweise substituiert sind durch ein oder mehrere von Halo, Hydroxy, Nitro oder einer Aminogruppe der Formel NRₕRⱼ, worin Rₕ und Rⱼ wie oben definiert sind, h) eine Gruppe der Formel O(CH₂)ₘR_{g}, in der m 2, 3, 4 oder 5 ist, und R_{g} stellt Hydroxy oder eine Gruppe der Formel NR_{d}R_{c} dar, in der R_{d} und R_{c} wie oben definiert sind; oder R_{g} stellt eine Gruppe der Formel CORₐ dar, worin Rₐ wie oben definiert ist und m ist 1, 2, 3, 4 oder 5, i) Nitro, j) wahlweise substituiertes Phenyl C₁₋₆ Alkyl, k) wahlweise substituiertes Phenyl C₁₋₆ Alkoxy, l) Cyano, m) eine C₃-6 Alkenyloxygruppe, n) eine Pyridyloxy- oder Pyridylthiogruppe, in der der Pyridinring wahlweise substituiert ist durch ein oder mehrere der folgenden: Trifluormethyl oder Nitro, o) Hydroxyamidino, p) Aminomethyl, q) Formamidomethyl, r) eine C₁₋₆ Alkylthiogruppe, s) Phenyl oder t) eine C₂₋₄ Alkenylgruppe oder eine C₂₋₄ Alkinylgruppe, wobei jede wahlweise substituiert ist durch Phenyl, das wahlweise substituiert ist durch ein oder mehrere der folgenden: eine C₁₋₆ Alkylgruppe, eine C₁₋₆ Alkoxygruppe oder Halo.

2. Verbindungen gemäß Anspruch 1, in denen X CH₂ oder S ist.

3. Verbindungen gemäß Anspruch 1, in denen X CH₂ ist und A ist NR₂₅.

4. Verbindungen gemäß Anspruch 1, in denen X S ist und A ist NR₂₅.

5. Verbindungen gemäß Anspruch 1, in denen X CH₂ ist und A ist HNSO₂.

6. Verbindungen gemäß Anspruch 1, in denen X CH₂ ist und A ist SO₂NH.

7. Verbindungen gemäß Anspruch 1, in denen X CH₂ ist und A ist CONH.

8. Verbindungen gemäß Anspruch 1, in denen X CH₂ ist und A ist HNCO.

9. Verbindungen gemäß Anspruch 1, in denen X CH₂ ist, A ist NR₂₅, L₁ ist (CH₂)_{q}, worin q eine ganze Zahl von 1 bis 6 ist und die Alkylenkette ist wahlweise substituiert durch ein oder mehrere der folgenden: eine C₁-₆ Alkylgruppe wahlweise substituiert durch ein oder mehrere von Hydroxy, Halo oder wahlweise substituiertes Amino; eine C₁₋₆ Alkoxygruppe wahlweise substituiert durch ein oder mehrere von Hydroxy, Halo oder wahlweise substituiertem Amino; Hydroxy; Halo; oder wahlweise substituiertes Amino; L₂ ist eine Bindung und R₁ ist wahlweise substituiertes Pyridyl.

10. Eine Verbindung gewählt aus:
4-(1,4-Dihydroindeno[1,2-c]pyrazol)-N-(4-pyridyl)benzylamin
N-[4-(1,4-Dihydroindeno[1,2-c]pyrazol-3-yl)phenyl]benzensulfphonamid
4-(1,4-Dihydroindeno[1,2-c]pyrazol-3-yl)-N-(2-methoxyethyl)benzamid
4-(1,4-Dihydroindeno[1,2-c]pyrazol-3-yl)-N-(4-nitrophenyl)benzamid
4-(1,4-Dihydroindeno[1,2-c]pyrazol-3-yl)imidazol-1-ylacetanilid
4-(1,4-Dihydroindeno[1,2-c]pyrazol-3-yl)-N-[2-(imidazol-1-yl)ethyl]anilin
4-(1,4-Dihydroindeno[1,2-c]pyrazol-3-yl)-N-(2-morpholinethyl)benzensulphonamid
4-(1,4-Dihydroindeno[1,2-c]pyrazol-3-yl)-N-(2-methoxyethyl)benzensulfphonamid
N-[2-(N,N-Diethylamino)ethyl]-4-(1,4-dihydroindeno[1,2-c]pyrazol-3-yl)benzylamin
N-[2-(N,N-Diethylamino)ethyl]-4-(1,4-dihydroindeno[1,2-c]pyrazol-3-yl)benzensulphonamid
4-(1,4-Dihydroindeno[1,2-c]pyrazol-3-yl)-N-(2-morpholinethyl)benzylamin
N-(4-Ethoxyphenyl)-4-(1,4-dihydroindeno[l,2-c]pyrazol-3-yl)benzylamin
(S)-4-(1,4-Dihydroindeno[1,2-c]pyrazol-3-yl)-N-(pyrrolidin-2-ylmethyl)benzamid
4-(1H-[1]Benzothieno[3,2-c]pyrazol-3-yl)-N-[3-(imidazol-lyl)propyl]benzylamin
4-(1H-[1]Benzothieno[3,2-c]pyrazol-3-yl)-N-(2-morpholinoethyl)benzylamin
und pharmazeutisch verträgliche Salze davon einschließlich einzelner Enantiomere und einer Mischung von Enantiomeren.

11. Eine pharmazeutische Zusammensetzung, umfassend eine Verbindung der Formel I, wie in Anspruch 1 definiert, in Verbindung mit einem pharmazeutisch verträglichen Verdünnungsmittel oder Träger.

12. Eine Verbindung gemäß Formel I, wie in Anspruch 1 definiert, für die Verwendung als ein Medikament.

13. Eine Verbindung gemäß Formel I, wie in Anspruch 1 definiert, für die Verwendung als ein Medikament zum Hemmen der Proteinkinaseaktivität.

14. Die Verwendung einer Verbindung gemäß Formel I, wie in Anspruch 1 definiert, in der Herstellung eines Medikaments zum Hemmen der Proteinkinaseaktivität.

15. Die Verwendung gemäß Anspruch 14, worin die Proteinkinase eine Tyrosinkinase ist.

16. Die Verwendung gemäß Anspruch 15, worin die Tyrosinkinase entweder eine Rezeptortyrosinkinase oder eine Nicht-Rezeptortyrosinkinase ist.

17. Die Verwendung gemäß Anspruch 16, worin die Tyrosinkinase gewählt ist aus der Gruppe bestehend aus KDR, flt-1, TIE-2, Lck, Src, fyn und yes.

18. Die Verwendung gemäß Anspruch 14, worin die Aktivität der Tyrosinkinase die Angiogenese beeinflußt.

19. Die Verwendung gemäß Anspruch 18, worin das Hemmen der Tyrosinkinase anti-angiogen ist.

20. Die Verwendung gemäß Anspruch 19, worin das Hemmen der Tyrosinkinase die Progression eines Krankheitsstatus hemmt, gewählt aus der Gruppe bestehend aus Krebs, Arthritis, Atherosklerose, Schuppenflechte (Psoriasis), Hämangiom, myokardiale Angiogenese, koronare und zerebrale kollaterale Vaskularisation, ischämische Gliederangiogenese, korneale Erkrankung, Hautrötung, neovaskuläres Glaukom, Makuladegeneration, Wundheilung, peptischer Ulkus, *Helicobacter* bezogene Krankheiten, Frakturen, diabetische Retinopathie und Katzenkratzkrankheit.

21. Die Verwendung gemäß Anspruch 15, worin die Aktivität der Tyrosinkinase die vaskuläre Hyperpermeabilität oder die Produktion von Ödemen beeinflußt.

22. Die Verwendung gemäß Anspruch 21, worin das Hemmen der Tyrosinkinase antiödematos ist.

23. Die Verwendung gemäß Anspruch 22, worin das Hemmen der Tyrosinkinase die Progression eines Krankheitsstatus hemmt, gewählt aus der Gruppe bestehend aus Verbrennungen, chronischer Lungenkrankheit, Anfall, Polypen, Schuppenflechte (Psoriasis), allergischer Entzündung, Makuladegeneration, diabetischer Retinopathie, ovarialem Hyperstimulationssyndrom und Gehirntumor-assoziiertem zerebralen Ödem.

24. Die Verwendung gemäß Anspruch 15, worin das Hemmen der Tyrosinkinase einen anti-Tumor-Effekt hat.

25. Die Verwendung gemäß Anspruch 15, worin das Hemmen der Tyrosinaktivität assoziiert ist mit anti-Fruchtbarkeits- oder Abtreibungsmitteleffekten.

26. Die Verwendung gemäß Anspruch 14, worin die Proteinkinase eine Serinkinase ist.

27. Die Verwendung gemäß Anspruch 14, worin die Proteinkinase eine Threoninkinase ist.

28. Die Verwendung gemäß Anspruch 15, worin die Tyrosinkinase KDR ist.

## Revendications

1. Composés de la formule I 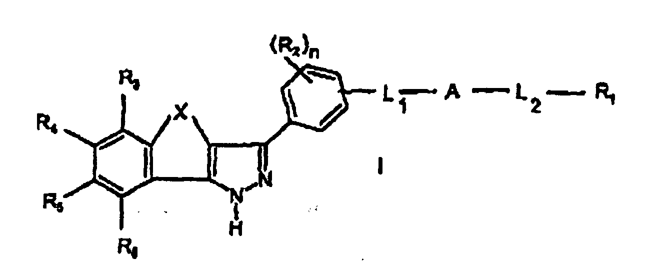 et leurs sels pharmaceutiquement acceptables dans lesquels
L₁ représente un groupe de la formule (E)ₛ(CH₂)_{q} dans laquelle E représente du NR₂₄, O ou S, a vaut 0 ou 1 et q est un nombre entier de 0 à 6, à condition que lorsque a vaut 1, q soit d'au moins 1, dans lequel la chaîne d'alcylène est éventuellement substituée par un ou plusieurs des éléments suivants : un groupe d'alkyle en C₁₋₆ substitué par un ou plusieurs groupes hydroxy, halo ou amino éventuellement substitué ; un groupe alkoxy en C₁₋₆ éventuellement substitué par un ou plusieurs groupes hydroxy, halo ou amino éventuellement substitué ; un groupe hydroxy, halo ou amino éventuellement substitué ;
A représente CONH, NHCO, SO₂NH, NHSO₂ ou NR₂₅ ;
L₂ représente un groupe de la formule (CH₂)ᵣ dans lequel r est un nombre entier de 0 à 6 dans lequel la chaîne d'alcylène est éventuellement substituée par un ou plusieurs des éléments suivants : un groupe alkyle en C₁₋₆ éventuellement substitué par un ou plusieurs groupes hydroxy, halo ou amino éventuellement substitués ; un groupe alkoxy en C₁₋₆ éventuellement substitué par un ou plusieurs groupes hydroxy, halo ou amino éventuellement substitué ; un groupe hydroxy, halo ou amino éventuellement substitué ;
R₂ représente un groupe alkyle en C₁₋₆ éventuellement substitué par un ou plusieurs des éléments suivants : un groupe halo, hydroxy, alkoxy en C₁₋₆ ou amino éventuellement substitué, un groupe alkoxy en C₁₋₆ éventuellement substitué par un ou plusieurs des éléments suivants : un groupe halo, hydroxy, alkoxy en C₁₋₆ ou éventuellement substitué ; ou R₂ est un groupe halo, hydroxy, cyano, nitro, carbamoyle, alkanoyle en C₁₋₆, un groupe carbonyl(alkoxy en C₁₋₅) ou un groupe amino éventuellement substitué ;
n représente 0, 1, 2 ou 3
X représente a) du méthylène substitué, b) du carbonyle, c) de l'oxygène, d) un groupe de la formule C=NOR₇ dans lequel R₇ représente H ou un groupe alkyle en C₁₋₄, e) un groupe de la formule NR₈ dans lequel R₈ représente H, un groupe alkyle en C₁₋₄ éventuellement substitué, ou un phényle éventuellement substitué, f) un groupe de la formule (CH₂)ₙ dans lequel n vaut 1, 2 ou 3 ou g) un groupe de la formule S(O)ₚ dans lequel p vaut 0, 1 ou 2.
R₃, R₄, R₅ et R₆ représentent indépendamment a) H, b) un groupe halo, c) un groupe alkyle en C₁₋₆ éventuellement substitué par un ou plusieurs des éléments suivants : hydroxy ; un groupe alkoxy en C₁₋₆ ; un groupe halo ;ou un groupe amino éventuellement substitué à condition que ces groupes ne soient pas fixés au carbone qui est fixé à l'oxygène du groupe alkoxy ; e) un groupe phénoxy éventuellement substitué, f) un hydroxy, g) une formule de groupe CORₐ dans laquelle Rₐ représente un hydroxy, un groupe alkoxy en C₁₋₆ ou Rₐ représente un groupe amino éventuellement substitué, h) un groupe amino éventuellement substitué i) un groupe alkanoyle en C₁₋₆, j) un groupe nitro, k) un alkyle en C₁₋₆ et phényle éventuellement substitué, l) un alkoxy en C₁₋₆ et phényle éventuellement substitué ; k) un alkyle en C₁₋₆ et phényle éventuellement substitué ; l) un alkoxy en C₁₋₆ et phényle éventuellement substitué ; m) un groupe cyano ou o) un groupe alcényle en C₂₋₄ ou un groupe alcynyle en C₂₋₄ ,
chacun d'eux étant éventuellement substitué par un phényle qui est éventuellement substitué par un ou plusieurs des éléments suivants : un groupe alkyle en C₁₋₆, un groupe alkoxy en C₁₋₆ ou un groupe halo ;
Et
1) quand A est du SO₂NH ou NHSO₂
R₁ représente a) un phényle éventuellement substitué b) un hétéroaryle éventuellement substitué, c) un anneau hétérocyclique saturé à cinq, six ou sept éléments contenant un atome d'azote qui contient éventuellement un atome hétéro supplémentaire choisi parmi O, S ou N et est éventuellement substitué par un groupe alkyle en C₁₋₆ dans lequel ledit anneau saturé peut être fixé à travers le carbone ou un atome hétéro d) un groupe amino éventuellement substitué ou e) un groupe alkoxy en C₁₋₆.
2) quand A représente CONH ou NHCO
R₁ représente a) un phényle substitué par un nitro ou un ou plusieurs groupes alkoxy en C₁₋₆ éventuellement substitués par un ou plusieurs des éléments suivants : groupe halo, hydroxy, alkoxy en C₁₋₆ ou amino éventuellement substitué b) un hétéroaryle éventuellement substitué ou c) un anneau hétérocyclique saturé à cinq, six ou sept éléments contenant un atome d'azote qui contient éventuellement un atome hétéro supplémentaire choisi parmi O, S ou N et est éventuellement substitué par un groupe alkyle en C₁₋₆ dans lequel ledit anneau saturé est fixé à travers un atome de carbone ou d) un groupe alkoxy en C₁₋₆ ;
3) Quand A représente un groupe NR₂₅ et q vaut au moins 1, alors
R₁ représente a) un phényle éventuellement substitué ; b) un hétéroaryle éventuellement substitué ; ou c) un groupe amino éventuellement substitué ; et
4) Quand A représente un groupe NR₂₅ et q vaut 0, et s vaut 0, alors R₁ représente un hétéroaryle éventuellement substitué ;
R₂₄ et R₂₅ représentent indépendamment H ; un groupe alkyle en C₁₋₆ éventuellement substitué par un ou plusieurs des éléments suivants : un groupe alkoxy en C₁₋₆, un groupe halo ou un groupe amino éventuellement substitué ; un groupe alkanoyle en C₁₋₆ ou un groupe d'alkylsulfonyle en C₁₋₆ ; à condition qu'aucun atome hétéro ne soit fixé au même atome de carbone hybridé sp3 ; dans lequel le méthylène substitué représente le méthylène substitué par un ou plusieurs de groupe hydroxy ou un alkyle en C₁₋₄ dans lequel le groupe alkyle est éventuellement ultérieurement substitué par un groupe de la formule NRᵣRₛ dans laquelle Rᵣ et Rₛ représentent indépendamment un H ou un groupe alkyle en C₁₋₆ ; Dans lequel le groupe éventuellement substitué représente un groupe de la formule NRₖRₗ dans laquelle Rₖ et Rₗ représentent indépendamment de l'hydrogène, un groupe alkyle en C₁₋₁₂, un groupe alkoxy en C₁₋₁₂, un groupe cycloalkyle en C₃₋₁₂, un groupe phényle, un groupe alkyle en C₁₋₆ phényle, un groupe hétéroaryle, ou un groupe alkyle en C₁₋₆ hétéroaryle, dans lequel chacun desdits groupes est éventuellement substitué par un ou plusieurs des éléments suivants : un groupe alkyle en C₁₋₆, un groupe alkoxy en C₁₋₆, un groupe hydroxy, halo ; ou Rₖ et R₁ ensemble avec l'atome d'azote auquel ils sont fixés représentent un anneau hétérocyclique saturé à cinq, six ou sept éléments qui contient éventuellement un atome hétéro supplémentaire choisi parmi O, S ou N et est éventuellement substitué par un groupe alkyle en C₁₋₆ ; Dans lequel l'hétéroaryle représente un hétérocycle aromatique mono ou bicyclique éventuellement substitué dans lequel l'hétérocycle contient 1, 2, 3 ou 4 hétéro-atomes choisis parmi l'azote, le soufre ou l'oxygène ; Dans lequel le phényle éventuellement substitué et
l'hétéroaryle représentent le phényle ou l'hétéroaryle substitués par un ou plusieurs des éléments suivants : a) halo, b) un groupe alkyle en C₁₋₆ éventuellement substitué par un ou plusieurs des groupes hydroxy, halo, amino éventuellement substitué ou un anneau hétérocyclique saturé à cinq, six ou sept éléments contenant un atome d'azote qui contient éventuellement un atome hétéro supplémentaire choisi parmi O, S ou N et est éventuellement substitué par un groupe alkyle en C₁₋₆ substitué par un ou plusieurs des éléments hydroxy, groupe alkoxy en C₁₋₆, halo ou un groupe amino éventuellement substitué à condition que ces groupes ne soient pas fixés au carbone qui est fixé à l'oxygène du groupe alkoxy, ou un anneau hétérocyclique saturé à cinq, six ou sept éléments contenant un atome d'azote qui contient éventuellement un atome hétéro supplémentaire choisi parmi O, S ou N et est éventuellement substitué par un groupe alkyle en C₁₋₆, dans lequel ledit anneau saturé est fixé à travers un atome de carbone ; d) un phénoxy éventuellement substitué , e)un hydroxy, f) un groupe de formule CORₐ dans lequel Rₐ représente un hydroxy, un groupe alkoxy en C₁₋₆ ou Rₐ représente un groupe de la formule NR_{b}R_{c} dans lequel R_{b} et R_{c} représentent indépendamment de l'hydrogène, un groupe alkyle en C₁₋₆ ou Rₐ représente un groupe de la formule NR_{b}R_{c} dans laquelle R_{b} et R_{c} représentent indépendamment de l'hydrogène, un groupe alkyle en C₁₋₁₂, un groupe cycloalkyle en C₃₋₁₂ ou un phényle dans lequel le groupe alkyle, le groupe cycloalkyle et le phényle sont éventuellement substitués par un ou plusieurs des hydroxy, halo, groupe cycloalkyle en C₃₋₁₂ ou un groupe amino de la formule NRₕRⱼ dans lequel Rₕ et Rⱼ représentent indépendamment de l'hydrogène ou un groupe alkyle en C₁₋₆ ou dans lequel Rₕ et Rⱼ ensemble avec l'atome d'azote auquel ils sont fixés représentent un anneau hétérocyclique saturé à cinq, six ou sept éléments qui contient éventuellement un atome hétéro supplémentaire choisi parmi O, S ou N et est éventuellement substitué par un groupe alkyle en C₁₋₆, g) un groupe de la formule NR_{d}Rₑ dans lequel R_{d} et Rₑ sont indépendamment choisis à partir d'hydrogène, un groupe alkyle en C₁₋₁₂, un groupe cycloalkyle en C₃₋₁₂ ou un phényle ou un groupe de la formule COR_{f} dans lequel R_{f} représente de l'hydrogène, un groupe alkyle en C₁₋₁₂ ; un groupe cycloalkyle en C₃₋₁₂, un groupe alkyle en C₁₋₆ phényle ou un phényle dans lequel dans chaque cas le groupe alkyle, le groupe cycloalkyle et le phényle sont éventuellement substitués par un ou plusieurs des groupes halo, hydroxy, nitro ou amino de la formule NRₕRⱼ dans laquelle Rₕ et Rⱼ sont comme définis ci-dessus, h) un groupe de la formule O(CH₂)ₘ R_{g} dans laquelle m vaut 2, 3, 4 ou 5 et R_{g} représente un hydroxy ou un groupe de la formule NR_{d}Rₑ dans laquelle R_{d} et Rₑ sont comme définis ci-dessus ; ou R_{g} représente un groupe de la formule CORₕ dans laquelle Rₕ est comme défini ci-dessus et m vaut 1, 2, 3, 4 ou 5, i) un nitro, j) un alkyle en C₁₋₆ éventuellement substitué en phényle, k) un alkoxy en C₁₋₆ de phényle éventuellement substitué ; l) un cyano, m) un groupe alcényloxy en C₃₋₆, n) un groupe pyridyloxy ou pyridylthio dans lequel l'anneau de pyridine est éventuellement substitué par un ou plusieurs des éléments suivants : trifluorométhyle, ou nitro, o) hydroxyamidino, p) aminométhyle, r) un groupe alkylthio en C₁₋₆, s) phényle ou t) un groupe alcényle en C₂₋₄ ou un groupe alcynyle en C_{2-4,} chacun d'eux étant éventuellement substitué par du phényle qui est éventuellement substitué par un ou plusieurs des éléments suivants : un groupe alkyle en C₁₋₆, un groupe alkoxy en C₁₋₆ ou un halo.

2. Composés selon la revendication 1 dans lesquels X est un CH₂ ou S

3. Composés selon la revendication 1 dans lesquels X est un CH₂ et A est du NR₂₅.

4. Composés selon la revendication 1, dans lesquels X est un S et A est du NR₂₅

5. Composés selon la revendication 1 dans lesquels X est du CH₂ et A est du HNSO₂.

6. Composés selon la revendication 1 dans lesquels X est du CH₂ et A est du SO₂NH

7. Composés selon la revendication 1 dans lesquels X est du CH₂ et A est du CONH

8. Composés selon la revendication 1 dans lesquels X est du CH₂ et A est du NHCO.

9. Composés selon la revendication 1, dans lesquels X est du CH₂, A est du NR₂₅, L₁ est du (CH₂)_{q} dans lequel q est un nombre entier de 1 à 6 et la chaîne d'alcylène est éventuellement substituée par un ou plusieurs des éléments suivants : un groupe alkyle en C₁₋₆ éventuellement substitué par un ou plusieurs groupes hydroxy, halo ou amino éventuellement substitués ; un groupe alkoxy en C₁₋₆ éventuellement substitué par un ou plusieurs groupes hydroxy, halo ou amino éventuellement substitués ; hydroxy, halo ou amino éventuellement substitué ; L₂ est une liaison et R₁ est un pyridyle éventuellement substitué.

10. Composé choisi parmi :
4-(1,4-dihydroindéno[1,2-c]pyrazole)-N-(4-pyridyl) benzylamine
N-[4-(1,4-dihydroindéno[1,2-c]pyrazol-3-yl)phényl]benzène sulfonamide
4-(1,4-dihydroindéno[1,2-c]pyrazol-3-yl)-N-(2-méthoxyéthyl)benzamide
4-(1,4-dihydroindéno[1,2-c]pyrazol-3-yl)-N-(4-nitrophényl) benzamide
4-(1,4-dihydroindéno[1,2-c]pyrazol-3-yl)imidazol-1-ylacétanilide
4-(1,4-dihydroindéno[1,2-c]pyrazol-3-yl)-N-[2-(imidazol-1-yl)éthyl]aniline
4-(1,4-dihydroindéno[1,2-c]pyrazol-3-yl)-N-(2-morpholinoéthyl)benzènesulfonamide
4-(1,4-dihydroindéno[1,2-c]pyrazol-3-yl)-N-(2-méthoxyéthyl)benzènesulfonamide
N-[2-(N,N-diéthylamino)éthyl]-4-(1,4-dihydroindéno[1,2-c]pyrazol-3-yl)benzylamine
N-[2-(N,N-diéthylamino)éthyl]-4-(1,4-dihydroindéno[1,2-c]pyrazol-3-yl)benzènesulfonamide
4-(1,4-dihydroindéno[1,2-c]pyrazol-3-yl)-N-(2-morpholinoéthyl)benzylamine
N-(4-éthoxyphényl)-4-(1,4-dihydroindéno[1,2-c]pyrazol-3-yl)benzylamine
(S)-4-(1,4-dihydroindéno[1,2-c]pyrazol-3-yl)-N-(pyrrolidin-2-ylméthyl)benzamide
4- (1H- [1]benzothiéno[3,2-c]pyrazol-3-yl) -N- [3- (imidazol-1-yl)propyl]benzylamine
4- (1H- [1]benzothiéno[3,2-c]pyrazol-3-yl)-N-(2-morpholinoéthyl)benzylamine
et leurs sels pharmaceutiquement acceptables, y compris les énantiomères individuels et les mélanges d'énantiomères.

11. Composition pharmaceutique contenant un composé de la formule 1, comme défini dans la revendication 1 en combinaison avec un diluant ou support pharmaceutiquement acceptable.

12. Composé de la formule I, comme défini dans la revendication 1 à utiliser comme médicament.

13. Composé de la formule I, comme défini dans la revendication 1, à utiliser comme médicament pour inhiber l'activité de protéine-kinase.

14. Utilisation d'un composé de la formule I, comme défini dans la revendication 1 dans la fabrication d'un médicament pour inhiber l'activité de la protéine-kinase.

15. Utilisation selon la revendication 14, dans laquelle ladite protéine-kinase est une tyrosine-kinase

16. Utilisation selon la revendication 15, dans laquelle ladite tyrosine-kinase est soit une tyrosine-kinase réceptrice, soit une tyrosine-kinase non réceptrice.

17. Utilisation selon la revendication 16, dans laquelle ladite tyrosine-kinase est choisie dans le groupe composé de KDR, flt-1, TIE-2, Lck, Src, fyn et yes.

18. Utilisation selon la revendication 14, dans laquelle l'activité de ladite tyrosine-kinase affecte l'angiogénèse.

19. Utilisation selon la revendication 18, dans laquelle l'inhibition de ladite tyrosine-kinase est anti-angiogénique.

20. Utilisation selon la revendication 19, dans laquelle ladite inhibition de ladite tyrosine-kinase inhibe la progression d'une maladie choisie dans le groupe composé de cancer, arthrite, athérosclérose, psoriasis, hémangiome, angiogénèse du myocarde, vascularisation collatérale cérébrale et coronarienne, angiogénèse ischémique des membres, maladie cornéenne, rubéose, glaucome néovasculaire, dégénérescence maculaire, cicatrisation, ulcère gastro-intestinal, maladies liées à l'Helicobacter, fractures, rétinopathie diabétique, et lymphoréticulose bénigne d'inoculation.

21. Utilisation selon la revendication 15, dans laquelle l'activité de ladite tyrosine-kinase affecte l'hyperperméabilité vasculaire ou la production d'oedèmes.

22. Utilisation selon la revendication 21, dans laquelle l'inhibition de ladite tyrosine-kinase est anti-oedémateuse.

23. Utilisation de la revendication 22, dans laquelle l'inhibition de ladite tyrosine-kinase inhibe la progression d'une maladie choisie dans le groupe composé de brûlures, maladie pulmonaire chronique, ictus, polypes, psoriasis, inflammation allergique, dégénérescence maculaire, rétinopathie diabétique, syndrome de l'hyperstimulation ovarienne et oedème cérébral associé à une tumeur au cerveau.

24. Utilisation selon la revendication 15 dans laquelle l'inhibition de ladite tyrosine-kinase a un effet anti-tumeur.

25. Utilisation selon la revendication 15, dans laquelle l'inhibition de ladite activité de la tyrosine est associée à des effets anti-fertilité ou abortifs.

26. Utilisation selon la revendication 14 dans laquelle ladite protéine-kinase est une sérine-kinase

27. Utilisation selon la revendication 15 dans laquelle ladite protéine-kinase est une thréonine-kinase

28. Utilisation selon la revendication 15 dans laquelle ladite tyrosine kinase est la KDR.
